# EUROPEAN PATENT SPECIFICATION

(11) **EP 3 092 944 B1**
(45) Date of publication and mention of the grant of the patent: **01.04.2020**
(21) Application number: 16169175.3
(22) Date of filing: 11.05.2016
(51) Int. Cl.: A61B 6/12, A61B 34/20, A61B 5/046, A61B 5/0464, A61B 5/055, A61B 5/0428, A61B 5/00, A61B 5/06, A61B 90/00, A61N 1/06, A61B 6/03, A61B 6/00, A61B 8/08, A61B 8/00, A61N 7/02, A61B 18/00

(54) **COMBINED ELECTROPHYSIOLOGICAL MAPPING AND CARDIAC ABLATION SYSTEMS**
KOMBINIERTE ELEKTROPHYSIOLOGISCHE MESSUNGEN UND HERZABLATIONSSYSTEME
SYSTÈMES DE CARTOGRAPHIE ÉLECTROPHYSIOLOGIQUE ET D'ABLATION CARDIAQUE COMBINÉS

(30) Priority: 13.05.2015 US 201562161049 P; 01.05.2016 US 201615143603
(43) Date of publication of application: 16.11.2016
(73) Proprietor: EP Solutions SA, 1400 Yverdon-les-Bains (CH)
(72) Inventor: KUCK, Karl-Heinz, 20249 Hamburg (DE); HALLER, Markus, 1260 Nyon (CH); STROEBEL, Joerg, 90482 Nürnberg (DE); MAIER, Michael, 1474 Chables (CH); TSIKLAURI, Mikhail, 121059 Moscow (RU); BUTSCHEIDT, Michael, 8832 Wilen b. Wollerau (CH); SCHULZE, Walther, 69120 Heidelberg (DE)
(74) Representative: Studio Torta S.p.A.

(56) References cited:
- WO-A1-2005/087128
- WO-A1-2015/063246
- US-A1- 2009 080 610
- US-A1- 2013 116 681
- US-A1- 2013 184 697
- US-A1- 2014 022 250
- US-A1- 2014 088 395
- US-A1- 2014 200 429
- US-A1- 2015 042 646

## Description

### Field of the Invention

Various embodiments described herein relate to the field of electrophysiological mapping and ablation medical systems, devices, components, and methods.

### Background

Cardiac ablation is typically an invasive medical procedure that is commonly used to treat many different types of cardiac arrhythmia, and usually involves advancing one or more catheters through a patient's blood vessels by means of percutaneous access to the patient's heart. An external ablation system provides energy (e.g., radiofrequency currents, laser radiation) or causes low-temperature exposure, through the ablation catheter to the endocardium or myocardium. The energy or low temperature destroys small areas of the heart tissue where cardiac arrhythmias are determined to originate.

It is often difficult to monitor the progress of a cardiac ablation procedure, or to determine the degree of success that has been achieved during the ablation procedure. Electrophysiology (EP) catheters can be employed in conjunction with ablation catheters to monitor electrical activity of the heart during and after the ablation procedure. Such procedures are, however, invasive, and typically require the use of multiple catheters and other invasive devices such as electrode baskets.

What is needed are improved methods and means of monitoring a patient's heart's electrical activity during a cardiac ablation procedure. What is also needed are methods and means for carrying out cardiac ablation procedures controllably, accurately, and with risks that are lower when compared to presently employed invasive cardiac ablation procedures.

US 2014/022250 A1 discloses a system for patient-specific planning and guidance of an ablation procedure for cardiac arrhythmia. A patient-specific anatomical heart model is generated based on pre-operative cardiac image data. The patient-specific anatomical heart model is registered to a coordinate system of intra-operative images acquired during the ablation procedure. One or more ablation site guidance maps are generated based on the registered patient-specific anatomical heart model and intra-operative patient-specific measurements acquired during the procedure.

US 2014/088395 A1 discloses an electrogram reconstruction method to generate reconstructed electrogram signals for each of a multitude of points residing on or near a predetermined cardiac envelope based on geometry data and non-invasively measured body surface electrical signals. The method can include a phase calculator to compute phase signals for the multitude of points based on the reconstructed electrogram signals and a visualization engine to generate an output based on the computed phase signals.

WO 2015/063246 A1 discloses a system for determining electrical potentials on a surface of a heart. An esophageal electrode structure measures electrical characteristics within an esophagus and a position determination unit determines the position of the esophageal electrode structure within the esophagus and the position of the surface of the heart. The electrical characteristics on the surface of the heart are then determined based on the measured electrical characteristics and based on the determined positions of the esophageal electrode structure and the surface of the heart.

US 2015/042646 A1 discloses a method for patient-specific planning and guidance of electrophysiological interventions. A patient-specific anatomical heart model is generated from cardiac image data of a patient. A patient-specific cardiac electrophysiology model is generated based on the patient-specific anatomical heart model and patient-specific electrophysiology measurements. Virtual electrophysiological interventions are performed using the patient-specific cardiac electrophysiology model. A simulated electrocardiogram (ECG) signal is calculated in response to each virtual electrophysiological intervention.

US 2013/184697 A1 discloses a method for non-invasive treatment of cardiac arrhythmias. The method includes acquiring body surface electrical signals at locations on a body surface of a living being from electrodes placed on locations of the body surface, reconstructing three-dimensional heart and torso anatomical models of the living being from an imaging scan, and calculating an electrical activity throughout three-dimensional volume of the heart by electrocardiogram inverse problem solving based at least in part on the acquired body surface electrical signals and the reconstructed three-dimensional heart and torso anatomical models.

### Summary

The invention is as specified in the claims.

In one embodiment of the present disclosure, there is provided a system for combined electrophysiological mapping and ablation of a patient's heart comprising an external electrophysiological mapping system (EMS) comprising: (a) a plurality of surface electrical sensing electrodes configured to acquire surface electrocardiogram (ECG) signals from at least portions of a patient's torso; (b) a data acquisition device operably connected to the surface electrical sensing electrodes and configured to condition the ECG signals provided thereby; (c) at least one non-transitory computer readable medium storing instructions executable by at least one processor to perform a method for receiving and processing the ECG signals to provide on a display or monitor a real-time or near-real-time voxel-model-derived visual representation or image of at least a portion of the patient's heart during a combined electrophysiological mapping and cardiac ablation procedure carried out on the patient, a cardiac ablation system comprising a catheter configured for insertion inside the heart of the patient, the catheter comprising a distal end comprising a tissue ablation device configured to controllably form scar tissue on the patient's endocardium during the combined electrophysiological mapping and cardiac ablation procedure, wherein the EMS is further programmed and configured to process the ECG signals during the combined electrophysiological mapping and cardiac ablation procedure to produce on the display or monitor the real-time or near-real-time voxel-model-derived visual representation of one or more locations on the patient's heart where at least one scar has been created by the ablation device during the combined electrophysiological mapping and cardiac ablation procedure.

The visual representation of the scarring location may be based upon a velocity field or gradient or an amplitude field or gradient, the field or gradient being calculated by the EMS. The cardiac ablation system may further comprise an electrical stimulation electrode located near or at the distal end of the catheter, the electrical stimulation electrode being configured to stimulate electrically intracardiac tissue of the patient to produce an evoked response therein, the EMS being configured to detect ECG signals corresponding to the evoked response and process such signals to provide or refine the visual representation of the intracardiac location where scarring created by the ablation device has occurred.
A location of the distal end of the catheter in the patient's heart may preferably be provided in the visual representation on the basis of a point of origin of the evoked response being calculated by the EMS.
The EMS and the CAS may together be configured to control a power level or duty cycle of the ablation delivered by the ablation device to the patient's heart, the power level or duty cycle being based on an amount, degree or extent of scarring of the patient's heart determined at least partially to have occurred by the EMS.
The EMS and the CAS may together be configured to control an amount of time ablation is delivered by the ablation device to the patient's heart, the amount of time being based on an amount, degree or extent of scarring of the patient's heart determined at least partially to have occurred by the EMS. The catheter may further comprise near or at its distal end at least one electrode, coil, sensor, transducer, magnetic source, or antenna that in combination with the EMS is configured to permit a location of the catheter's distal tip within the patient's heart to be determined and displayed on the monitor or display in real-time or near-real-time.
The catheter may further comprise near or at its distal end at least one electrical sensing electrode configured to sense electrical signals generated by the heart, the cardiac ablation system being configured to provide the electrical signals sensed thereby to the EMS as input signals thereto. The tissue ablation device may preferably be a cryogenic ablation device, a radiofrequency ablation device, an ultrasound ablation device, a high-intensity focused ultrasound device, a chemical ablation device, or a laser ablation device.

In another embodiment, there is provided a non-invasive system for combined electrophysiological mapping and ablation of a patient's heart comprising an external electrophysiological mapping system (EMS) comprising: (a) a plurality of surface electrical sensing electrodes configured to acquire surface electrocardiogram (ECG) signals from at least portions of a patient's torso; (b) a data acquisition device operably connected to the surface electrical sensing electrodes and configured to condition the ECG signals provided thereby; (c) at least one non-transitory computer readable medium storing instructions executable by at least one processor to perform a method for receiving and processing the ECG signals to produce on a display or monitor a real-time or near-real-time visual voxel-model-derived representation or image of at least a portion of the patient's heart during a combined electrophysiological mapping and cardiac ablation procedure carried out on the patient, an external non-invasive cardiac ablation system (CAS) comprising at least one external directionally controllable and focusable source of ablation energy, the ablation energy source being configured to controllably form scar tissue on the patient's endocardium during the combined electrophysiological mapping and cardiac ablation procedure, wherein the EMS is further programmed and configured to process the ECG signals during the combined electrophysiological mapping and cardiac ablation procedure to produce on the display or monitor a real-time or near-real-time visual representation of one or more locations on the patient's heart where at least one scar has been created by the ablation device during the combined electrophysiological mapping and cardiac ablation procedure.

The visual representation of the scarring location may be based upon a velocity field or gradient or an amplitude field or gradient, an electrical conductivity field or gradient, the field or gradient being calculated by the EMS.
The ablation energy source of the cardiac ablation system may be included in a high intensity focused ultrasound (HIFU) system, a proton beam radiotherapy system, or an X-ray beam radiotherapy system.
The combined non-invasive electrophysiological mapping and ablation system may further comprise a magnetic resonance imaging (MRI) and guiding system configured to a three-dimensional image of at least a portion of the patient's heart and to guide the location of the ablation energy that is applied to the patient's heart during the combined electrophysiological mapping and cardiac ablation procedure.
The MRI and guiding system and the EMS may together be configured to produce on the display or monitor the real-time or near-real-time visual representation or image of at least a portion of the patient's heart and the location of the ablation energy applied to the patient's heart.
The combined non-invasive electrophysiological mapping and ablation system may further comprise a computer tomography (CT) imaging and guiding system configured to generate a three-dimensional image of at least a portion of the patient's heart and to guide the location of the ablation energy that is applied to the patient's heart during the combined electrophysiological mapping and cardiac ablation procedure.
The CT imaging and guiding system and the EMS may together be configured to produce on the display or monitor the real-time or near-real-time visual representation or image of at least a portion of the patient's heart and the location of the ablation energy applied to the patient's heart. The combined non-invasive electrophysiological mapping and ablation system may further comprise an ultrasound imaging and guiding system configured to generate a three-dimensional image of at least a portion of the patient's heart and to guide the location of the ablation energy that is applied to the patient's heart during the combined electrophysiological mapping and cardiac ablation procedure.

The ultrasound imaging and guiding system and the EMS may together be configured to produce on the display or monitor the real-time or near-real-time visual representation or image of at least a portion of the patient's heart and the location of the ablation energy applied to the patient's heart. The EMS and the CAS may together be configured to control a power level or duty cycle of the ablation delivered by the ablation device to the patient's heart, the power level or duty cycle being based on an amount, degree or extent of scarring of the patient's heart determined at least partially to have occurred by the EMS.
The EMS and the CAS may together be configured to control an amount of time ablation is delivered by the ablation device to the patient's heart, the amount of time being based on an amount, degree or extent of scarring of the patient's heart determined at least partially to have occurred by the EMS.

In still another embodiment, there is provided a method of visualizing on a monitor or display at least one location where scar tissue has been or is being formed in or on a patient's heart during a combined electrophysiological mapping and ablation procedure comprising acquiring, during the combined procedure, ECG signals from a surface of the patient's torso, processing, in a combined electrophysiological mapping and ablation system, the ECG signals, providing, on the monitor or display, a real-time or near-real-time visual representation or image of electrical activity occurring over at least a portion of the patient's heart during the combined procedure, ablating a portion of the patient's heart with an ablation device and forming scar tissue thereon or therein, continuing to process, in the combined electrophysiological mapping and ablation system, ECG signals acquired or being acquired from the surface of the patient's torso, providing, on the monitor or display, a real-time or near-real-time visual representation or image of one or more locations on the patient's heart where scar tissue has been formed or is being formed therein or thereon by the ablation device. The method may further comprise using spatial position data, the spatial position data being generated by an imaging system operably connected to or forming a portion of the combined electrophysiological mapping and ablation system, the spatial position data being based upon or related to the visual representation or image of the one or more locations where scar tissue has been formed or is being formed, the spatial position data being employed to control further positioning of the ablation device with respect to the patient's heart such that new scar tissue is formed therein or thereon in at least one desired new scar location.

Further embodiments are disclosed herein or will become apparent to those skilled in the art after having read and understood the specification and drawings hereof.

### Brief Description of the Drawings

Different aspects of the various embodiments will become apparent from the following specification, drawings and claims in which:
Fig. 1 shows one embodiment of a basic method and system 10 for combined electrophysiological mapping of a patient's heart activity and ablation of the patient's heart;
Fig. 2 shows one embodiment of a schematic block diagram of system 10;
Figs. 3A through 3E show various devices and components associated with one embodiment of mapping electrode system 100;
Figs. 4A through 4C show embodiments of electrophysiological mapping sensor patches;
Fig. 5A shows one embodiment of a data acquisition device or measurement system 210 of system 10;
Fig. 5B shows one embodiment of portions of interface cable box 240, MMU 200/250 and PVM 400/450;
Fig. 6 shows one embodiment of a method 602 for providing electrophysiological mapping results;
Fig. 7 shows another embodiment of a method 603 for providing electrophysiological mapping results;
Fig. 8 shows one embodiment of a method 601 for performing combined electrophysiological mapping and cardiac ablation;
Fig. 9A shows a schematic view of one embodiment of a method and devices employed to carry out invasive cardiac ablation;
Fig. 9B shows a schematic block diagram of one embodiment of a system configured to carry out invasive or non-invasive cardiac ablation procedures;
Fig. 9C shows a schematic block diagram of one embodiment of a system configured to carry out non-invasive cardiac ablation procedures;
Fig. 10(a) shows a partial cross-section view of a patient's heart with cardiac ablation and EP catheters disposed therein;
Figs. 10(b) through 10(f) illustrate various embodiments of visual representations and images generated by system 10 during the course of performing a combined electrophysiological mapping and ablation procedure;
Figs. 11(a) through 11(h) illustrate several different embodiments of algorithms that may be employed in system 10, and
Fig. 12 shows one embodiment of a computer system 700 that may be employed in system 10.

The drawings are not necessarily to scale. Like numbers refer to like parts or steps throughout the drawings.

### Detailed Descriptions of Some Embodiments

Described herein are various embodiments of systems, devices, components and methods for conducting combined electrophysiological mapping and ablation procedures.

Technical features described in the context of a specific embodiment may be combined with the features of other described embodiments unless the contrary is explicitly stated.

At least portions or components of the EP Solutions 01C System for Non-Invasive Cardiac Electrophysiology Studies (which is based upon and in most aspects the same as the AMYCARD 01 C System for Non-Invasive Cardiac Electrophysiology Studies) may be adapted for use in conjunction the various embodiments described and disclosed herein. Portions of the EP Solutions 01C System (hereafter "the EP Solutions 01C System") and other relevant components, devices and methods are described in: (a) U.S. Patent No. 8,388,547 to Revishvili et al. entitled "Method of Noninvasive Electrophysiological Study of the Heart" ("the '547 patent"); (b) U.S. Patent No. 8,529,461 to Revishvili et al. entitled "Method of Noninvasive Electrophysiological Study of the Heart" ("the '461 patent"), and (c) U.S. Patent No. 8,660,639 to Revishvili et al. entitled "Method of Noninvasive Electrophysiological Study of the Heart" ("the '639 patent").

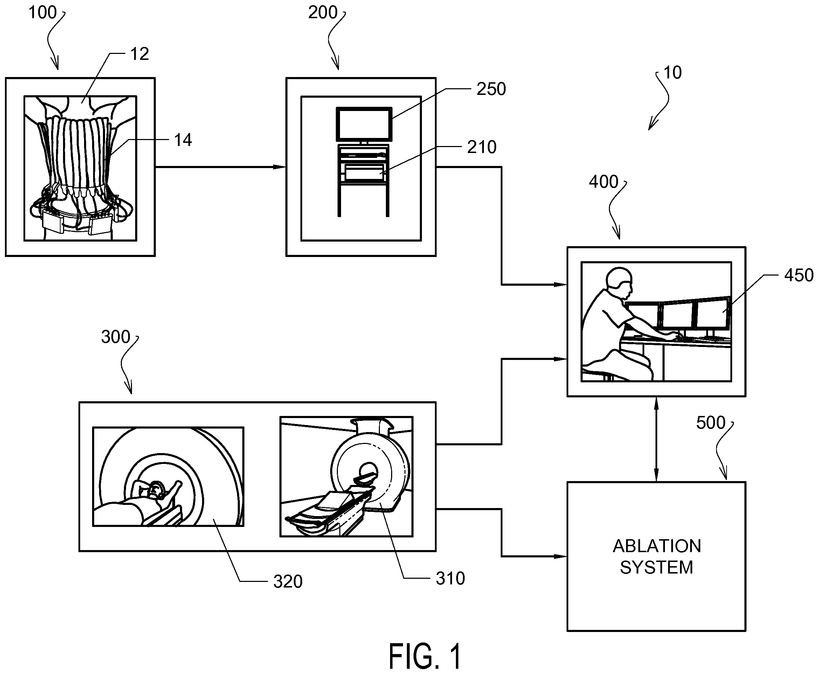

Referring now to Fig. 1, there is shown one embodiment of a basic method and system 10 for non-invasive electrophysiological mapping of a patient's heart activity while cardiac ablation is being carried out (and in some cases, after or shortly after, cardiac ablation has occurred). As shown, electrophysiological mapping system 10 ("EPM 10") comprises four basic sub-systems: (a) mapping electrode system 100 ("MES 100") disposed on patient 12's torso 14; (b) multichannel mapping unit 200 ("MMU 200"), which in one embodiment comprises a data acquisition device 210 and a corresponding first computer or computer workstation 250 for multichannel mapping of the heart; (c) scanner or imaging device 300, which in one embodiment is a computed tomography scanner 310 or an MRI scanner 320 (although other suitable types of medical imaging devices and systems may also be used, such as X-ray fluoroscopy); (d) processing and visualization module 400 ("PVM 400"), which in one embodiment comprises a second computer or computer workstation 450,and (e) ablation system 500, which may comprise a third computer or computer workstation 550. (Note that in some embodiments the first computer 250 of MMU 200, the second computer 450 of PVM 400,and even third computer 550 may be combined into a single computer or computer workstation, may comprise more than three computers or computer workstations, and/or may include computing and processing capability and/or storage provided by a network of local or remote computers, servers, and/or the cloud.)

In one embodiment, MES 100 comprises a plurality of electrical sensing electrodes E₁ ... Eₙ positioned on torso 14 of patient 12 (and in some embodiments on other portions of patient 12's body). Sensing electrodes in MES 100 are configured to sense electrical activity originating in patient 12's body. In addition to electrical sensing electrodes, other types of devices and/or transducers, such as ground electrodes, high intensity focused ultrasound transducers, ultrasound probes, navigation patches, cardioversion patches, and the like (more about which is said below), may be configured to operate in conjunction with, be incorporated into, or form a portion of MES 100 and/or system 10.

In one embodiment, and by way of non-limiting illustrative example, MES 100 comprises one or more of an ECG cable with 12 leads and corresponding electrodes, an ECG cable with 4 leads and corresponding electrodes, a patient cable for ECG-mapping with 8 contacts or electrodes, one or more special ECG-mapping cables (each with, for example, 56 contacts or electrodes), and special disposable or reusable mapping electrodes, each strip of disposable or reusable mapping electrodes having 8 contacts or electrodes. One example of a disposable ECG electrode is Model No. DE-CT manufactured by EP Solutions SA, Rue Galilee 7, CH-1400 Yverdon-les-Bains. Many different permutations and combinations of MES system 100 are contemplated having, for example, reduced, additional or different numbers of electrical sensing and other types of electrodes, sensors and/or transducers.

In one embodiment, MES 100 further comprises or operates in conjunction with sensors and/or transducers associated with monitoring and/or delivering an ablation therapy to the patient's heart, such as RF ablation catheters having one or more position transmitting coils or antennas located at or near the distal end thereof, which are configured to transmit high-frequency electromagnetic signals of high-intensity focused ultrasound (HIFU) transducers, ultrasound transducers, ultrasound receivers, ultrasound sensors, sensing electrodes, coils or inductors, electrical field sensors or transducers, and/or magnetic field sensors or transducers. In some embodiments, these sensors and/or transducers may also be configured to form portions of ablation system 500 .

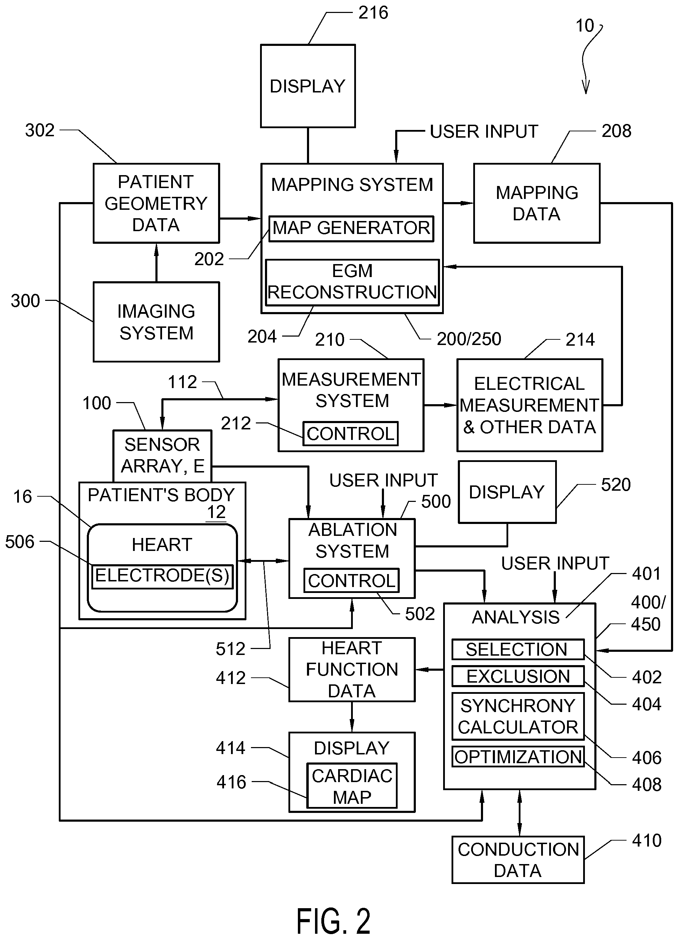

Scanner or imaging system 300 is used to help identify and determine the precise positions of the various electrodes included in MES 100 that have been placed in various positions and locations on patient 12's body, and is configured to provide patient geometry data 302 (see, for example, Fig. 2). Surface electrodes or position markers located on the patient's torso or in other locations on the patient's body can be configured to act as fiducial markers for imaging system 300.

In one embodiment, MES 100 further comprises or operates in conjunction with sensors and/or transducers associated with monitoring and/or delivering an ablation therapy to the patient's heart, such as high-intensity focused ultrasound (HIFU) transducers, ultrasound transducers, ultrasound receivers, ultrasound sensors, sensing electrodes, coils or inductors, electrical field sensors or transducers, and/or magnetic field sensors or transducers. In some embodiments, such sensors and/or transducers are configured to form portions of the ablation system 500.

In other embodiments, or in addition, such sensors and/or transducers are configured to provide inputs to a navigation or position/location determination system or device so that the spatial position of the ablation catheter within or on the heart, or the spatial location of the ablation therapy being delivered non-invasively to the patient's heart, may be determined. One catheter navigation system is described in U.S. Patent No. 6,947,788 entitled "Navigable catheter" to Gilboa et al., the entirety of which is hereby incorporated by reference herein, and which describes receiving and transmitting coils disposed in a catheter, and which permits the position of the catheter in a patient's body to be determined. The frequencies of transmitting and/or receiving coils or antennae in a catheter can be configured to operate outside the range of the frequencies of heart electrical signals to avoid or reduce the possibility of interference therewith (e.g., greater than 500 or 1,000 Hz).

Referring now to Figs. 1 and 2, ECG data are acquired from MES 100 by MMU 200, which in one embodiment comprises a data acquisition device or measurement system 210 that filters and amplifies analog signals provided by MES 100, digitizes such analog signals using one or more analog-to-digital converters ("ADCs") and associated processors or microprocessors, and sends or relays, or otherwise transfers or has transferred, the amplified and digitized signals to first computer or computer workstation 250. In one embodiment, data acquisition device 210 permits multichannel synchronous EKG/ECG recording from, by way of example, 240 or more surface electrodes positioned on a patient's skin and torso, as well as multichannel synchronous EKG/ECG recording from additional or other electrodes or channels (as described above in connection with MES 100).

In one embodiment, first computer or computer workstation 250 stores or records the amplified and digitized signals provided by data acquisition device 210. Signal digitization and recording functions can also be apportioned or split between data acquisition device 210 and first computer or computer workstation 250. Data from scanner or imaging system 300 and ECG data sensed by MES 100 and acquired and recorded by MMU 200 are then both input into PVM 400. In one embodiment, ECG data from patient 12 are acquired using MES 100 and data acquisition device 210 from unipolar electrodes positioned on patient's torso 14. The precise locations of such electrodes on patient's torso 14 are determined in PVM 400 using patient geometry data 302 provided by scanner or imaging system 300. (In other embodiments, patient geometry data 302 are calculated using input data from imaging system 300, in MMU 200, PVM 400, and/or ablation system 500. In still other embodiments, patient geometry data are provided to one or more of any of MMU 200, PVM 400 and ablation system 500.) ECG data recorded by MMU 200 may be stored on a CD, a USB memory stick, in RAM, on an electronic storage device such as a hard or flash drive, or in another memory device or component, and may then be exported or transferred to PVM 400 using such a storage device. Alternatively, ECG data recorded by MMU 200 may be transferred to PVM, by way of non-limiting illustrative example, using a local area network (LAN), a wide area network (WAN), wireless communication means (e.g., using Bluetooth or the Medical Implant Communication System or MICS), the internet or the cloud, or by suitable computer communication means known to those skilled in the art. In PVM 400, computed tomography of the chest and heart area is carried out, and processing and analysis of multichannel ECG data and computed tomography data are executed.

By way of non-limiting illustrative example, PVM 400 comprises a second computer or computer workstation 450 that comprises a specialized processing and visualization computer or series of interconnected computers or processors, which include pre-loaded and pre-programmed software configured to conduct electrophysiological studies. Second computer or computer workstation 450 typically comprises a keyboard, a mouse, a display 414 (such as a 24" or 25" LCD monitor), and a printer. PVM 400 and second computer or computer workstation 450 are configured for advanced mathematical processing of computed tomographic study data combined with multichannel ECG body surface mapping data, which together make it possible to perform computed non-invasive activation mapping of the patient's heart.

In some embodiments, and as mentioned above, MMU 200 and PVM 400 are combined in a single computing platform or computer workstation, and the functionality provided by the combination of MMU 200 and PVM 400 are combined into and provided by such a single computing platform or computer workstation. Increased computing performance for such a single computing platform can be provided by multiple processors arranged in parallel and increased RAM and ROM

Ablation system 500 is configured to operate in conjunction with one or more of MES 100, MMU 200, scanner or imaging system 300 and PVM 400, and provides ablation therapy to a patient's heart, either invasively or non-invasively, more about which is said below.

Together, MES 100, MMU 200, scanner 300, PVM 400 and ablation system 500 comprise EPM 10, and employ a technique known as NIEM (Non-Invasive Electrophysiological Mapping), which is an electrophysiological method based on non-invasive reconstruction of cardiac activation patterns sensed by a dense network of surface electrodes attached to the patient's torso. NIEM is employed in EPM 10 to permit non-invasive numerical reconstruction of endocardial and/or epicardial electrograms originating from the patient's ventricles and atria. Mathematical algorithms executed by EPM 10 are applied to the acquired unipolar surface ECG data to permit 3D reconstruction of the patient's heart and thorax.

In one embodiment, EPM 10 reconstructs electrograms using advanced tomographic techniques that eliminate the need to perform invasive surgical procedures on the patient's body, as described in the '547 patent, the '461 patent, and the '639 patent incorporated by reference herein above. Based on surface electrograms acquired on the patient's torso, time-varying electric field potentials of the patient's heart are calculated using tomographic techniques and algorithms. Actual boundaries of the patient's chest and lung surfaces, and of the patient's epicardial and endocardial heart surfaces, are determined by solving differential equation systems. Continuations of electric field potentials throughout the patient's chest surfaces and back to the patient's epicardial heart surfaces are implemented computationally based on a solution of the Cauchy problem for the Laplace equation in an inhomogeneous medium. When solving the Cauchy problem using the Laplace equation, a model of the chest is employed having tissues that lie within the bounds of large anatomic structures (e.g., the lungs, mediastinum, and/or spine), and that have constant coefficients of electroconductivity. Heart electric field potentials are assigned harmonic functions in each region, where each region has a constant coefficient of electroconductivity and satisfies conjugate conditions at the region's borders for electrical potential and current.

FIG. 2 depicts in further details one embodiment of a system 10 that can be utilized for assessing electrophysiologically the function of a patient's heart 16, and delivering and monitoring the ablation therapy provided to the patient's heart 16. System 10 can perform electrophysiological assessment of heart 16 in real time or near-real time as part of a diagnostic procedure and/or as part of a cardiac ablation therapy delivery procedure. In one embodiment, the ablation therapy is delivered invasively using, for example, a cardiac ablation catheter 512 and corresponding ablation system components. In another embodiment, the ablation therapy is delivered non-invasively. System 10 aids the physician or other health care provider in determining the parameters that should be used to deliver the ablation therapy to the patient (e.g., ablation therapy delivery location, and amount and type of ablation therapy).

In invasive ablation therapy delivery embodiments, and by way of non-limiting example, the ablation delivery therapy may be delivered by a cryogenic ablation device and/or system, a radiofrequency ablation device and/or system, an ultrasound ablation device and/or system, a high-intensity focused ultrasound (HIFU) device and/or system, a chemical ablation device and/or system, or a laser ablation device and/or system.

For example, in one embodiment, a cardiac ablation catheter 512 and/or an electrophysiological (EP) catheter having one or more stimulating (e.g., pacing) and/or sensing electrodes affixed thereto is inserted into the patient's body 12 so as to contact the patient's heart 16, either endocardially or epicardially. Those skilled in the art will understand and appreciate various type and configurations of cardiac ablation and/or stimulating and/or sensing catheters and/or EP catheters may be utilized to position the electrode(s) in the patient's body 12. In one embodiment, X-ray fluoroscopy is utilized to aid in determining the position of the catheter and its electrode(s) with respect to the patient's heart and elsewhere in the patient's body 12 as the catheter(s) is/are being delivered to the patient's heart, as well as during the ablation procedure.

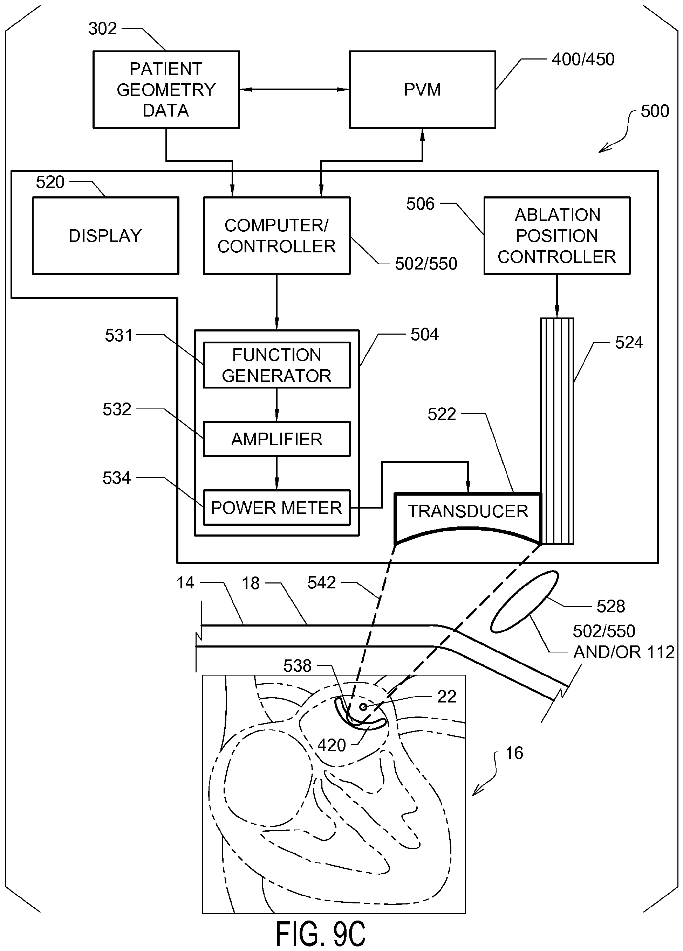

Ablation system 500 controls the ablation therapy delivered to the patient's heart 16. For instance, ablation system 500 may include control circuitry, a computer and/or a controller 502that can control the provision of RF signals via a conductive link electrically connected between the electrode(s) of catheter 512 and the ablation system 500. Control system 502 is configured to control ablation parameters (e.g., current, power level, duration of application of the ablation, time, voltage, duty cycle, pulse width, etc.) for applying the ablation therapy to the patient's endocardium or epicardium. Control system 502 can also control electrical sensing and stimulation parameters (e.g., current, voltage, impedance, temperature, repetition rate, trigger delay, sensing trigger amplitude) for applying electrical stimulation or for sensing electrical, temperature, impedance or other signals, via the electrode(s) incorporated into catheter 512. Control circuitry 502 can set ablation, stimulation and/or sensing parameters and apply the ablation therapy, stimulation and/or sensing parameters automatically or with user input, or by a combination of automatic and manual means. One or more sensors (e.g., sensor array of MES 100) and imaging system 300 (and patient geometry data 302) can also communicate sensor, navigational, or positional information to ablation system 500, which is located external to the patient's body 12. In one embodiment, the position of ablation catheter 512 and its electrodes inside or outside the patient's heart, or the location of the ultrasound or other type of ablation beam that is delivering ablation therapy inside or outside the patient's heart, can be determined and tracked via an imaging modality (e.g., any combination of MMU 200, PVM 400 and/or ablation system 500 working in combination with scanner or imaging system and patient geometry data 302), direct vision or the like. The location of ablation catheter 512 and/or its electrode(s), or the location of an ultrasound, particle or other type of ablation beam 544 (see, for example, Fig. 9C), and the therapy parameters associated therewith can be combined to provide corresponding therapy parameter and information and data regarding the progress of the ablation therapy as it is being delivered to the patient, or a short period of time thereafter

Concurrently with, or before or after, providing the ablation therapy via ablation system 500, system 10 is utilized to acquire electrophysiological information from the patient. In the example of FIG. 2, MES 100 comprising multiple surface electrodes is utilized to record patient electrophysiological activity. As described above, additional electrophysiological data may be acquired using electrical sensing/navigational/positional electrodes, coils or sensors incorporated into ablation system 500.

Alternatively or additionally, in other embodiments, MES 100 and/or ablation system 500 can comprise one or more invasive sensors, such as an EP catheter having a plurality of electrodes. The EP catheter can be inserted into the patient's body 12 and into heart 16 for mapping electrical activity for an endocardial surface, such as the wall of a heart chamber. In another embodiment, MES 100 can comprise an arrangement of sensing electrodes disposed on devices such as patches, which are placed on or near a patient's heart epicardially. These patches can be utilized during open chest and minimally invasive procedures to record electrical activity.

In each of such example approaches for acquiring patient electrical information, including by invasive or non-invasive means, or by a combination of invasive and non-invasive means, MES 100 and/or ablation system 500 provides the sensed electrical information to a corresponding measurement system such as measurement system or data acquisition device 210. The measurement system (e.g., data acquisition device 210) can include appropriate controls and signal acquisition and processing circuitry 212 for providing corresponding measurement or sensor data 214 that describes electrical activity detected by the sensors in MES 100 and/or ablation system 500. The measurement data 212 can include analog or digital information.

Data acquisition device ore measurement system 210 can also be configured to control the data acquisition process for measuring electrical activity and providing the measurement data. The measurement data 214 can be acquired concurrently with the delivery of ablation therapy by the ablation system, such as to detect electrical activity of the heart 16 that occurs in response to applying the ablation therapy (e.g., according to therapy delivery parameters). For instance, appropriate time stamps can be utilized for indexing the temporal relationship between the respective measurement data 214 and therapy parameters to facilitate the evaluation and analysis thereof.

MMU 200/250 is programmed to combine the measurement data 214 corresponding to electrical activity of heart 16 with patient geometry data 302 derived from scanner/imaging device 300 by applying an appropriate algorithm to provide corresponding electroanatomical mapping data 208. Mapping data 208 can represent electrical activity of the heart 16, such as corresponding to a plurality of reconstructed electrograms distributed over a cardiac envelope for the patient's heart (e.g., an epicardial envelope). As one example, mapping data 208 can correspond to electrograms for an epicardial or endocardial surface of the patient's heart 16, such as based on electrical data that is acquired non-invasively via sensors distributed on the body surface or invasively with sensors distributed on or near the epicardial or endocardial envelope. Alternatively, mapping data 208 can be reconstructed for an endocardial surface of a patient's heart such as a portion of chambers of the patient's heart (e.g., left and right ventricles, or left and right atria), such as based on electrical activity that is recorded invasively using an EP catheter or similar devices or recorded non-invasively via body surface sensors. The mapping data can represent electrical activity for other cardiac envelopes. The particular methods employed by the MMU 200/250 for reconstructing the electrogram data can vary depending upon the approach utilized for acquiring the measurement data 214. In addition, and as described further herein, the functionality of MMU 200/ 250 can be combined with any one or more of PVM 400/450, ablation system 500, and scanner or imaging system 300 to provide the data processing, analysis and display of electrophysiological and other data that have been or are being acquired from the patient.

In one example, MMU 200 generates mapping data 208 to represent activation times computed for each of the plurality of points on the surface of or inside the heart from electrograms over a selected cardiac interval (e.g., a selected beat). Since data acquisition device 210, and in some embodiments ablation system 500 can measure electrical activity of the heart concurrently, the resulting electrogram maps and activation maps (e.g., mapping data 208) thus can also represent concurrent data for the heart for analysis to quantify an indication of synchrony. The interval for which the activation times are computed can be selected based on user input. Additionally or alternatively, the selected intervals can be synchronized with the application of the ablation therapy by the ablation system 500.

In the example of Fig. 2, MMU 200 (which includes a mapping system) may comprise map generator 202 that constructs electroanatomical mapping data by combining measurement data 214 with patient geometry data 302 through an algorithm that reconstructs the electrical activity of the patient's heart 16 onto a representation (e.g., a three-dimensional representation) of the patient's heart 16. MMU 200 can also include an electrogram reconstruction engine 204 that processes the electrical activity to produce corresponding electrogram data for each of a plurality of identifiable points on the appropriate cardiac envelope (e.g., an epicardial or endocardial surface) of the patient's heart.

As an example, patient geometry data 302 may be in the form of graphical representation of the patient's torso, such as image data acquired from the patient using scanner/imaging device 300. Such image processing can include extraction and segmentation of anatomical features, including one or more organs and other structures, from a digital image set. Additionally, a location for each of the electrodes in sensor array 100 can be included in the patient geometry data 302, such as by acquiring the image while the electrodes are disposed on the patient and identifying the electrode locations in a coordinate system through appropriate extraction and segmentation. The resulting segmented image data can be converted into a two-dimensional or three-dimensional graphical representation that includes a region of interest for the patient.

Alternatively, patient geometry data can correspond to a mathematical model, such as a generic model of a human torso or a model that has been constructed based on image data acquired for the patient's heart 16. Appropriate anatomical or other landmarks, including locations for the electrodes in sensor array 100 can be identified in the patient geometry data 302 to facilitate registration of the electrical measurement data 214 and performing an inverse method thereon. The identification of such landmarks can be done manually (e.g., by a person via image editing software) or automatically (e.g., via image processing techniques).

By way of further example, the patient geometry data 302 can be acquired using nearly any imaging modality based on which a corresponding representation can be constructed. Such imaging may be performed concurrently with recording the electrical activity that is utilized to generate the patient measurement data 302 or the imaging can be performed separately (e.g., before the measurement data are acquired).

System 10 further includes PVM 400/450 that is configured and programmed to assess heart function and provide heart function data or visualizations based on the mapping data 208. As described herein, heart function data 412 may be in the form of an index or indices, or may be provided in the form of a two-dimensional or three-dimensional visual representation of the patient's heart's electrical activity. Additionally, and in some embodiments, PVM 400/450 can be configured to communicate with ablation system 400 and data acquisition device 210 so as to synchronize and control delivery of the ablation therapy and measurement of electrical activity via sensor array 100. PVM 400 can be configured to compute a plurality of indices or parameters according to different ablation therapy parameters (e.g., location of the ablation, sensing, and/or electrical stimulation parameters) based on the mapping data 208. PVM 400 may also be configured to compute heart histogram data, or to determine a desired (e.g., optimum) set of ablation therapy parameters for achieving desired therapeutic results. PVM 400 can also be configured to provide an indication of a patient's candidacy for ablation therapy, which may include one or both of an indication of the patient's expected responsiveness to ablation therapy or expected non-responsiveness to ablation therapy.

In the example of Fig. 2, PVM 400/450 may be configured and programmed to include a selection function 402, an exclusion function 404, a synchrony calculator 406 and an optimization component 408. The selection function 402 can be programmed to select an interval of a heart beat for which the analysis and heart function data will be calculated. The selection function 402 can be automated, such as synchronized to application of the ablation therapy via the ablation system 500. Alternatively, the selection function 402 can be manual or semiautomatic to permit selection of one or more cardiac intervals.

Exclusion/Inclusion function 404 may be programmed to identify and exclude, or to include, certain areas of the patient's heart from analysis, such as scar or scar formation areas, or certain chambers or other portions of the patient's heart 16. The exclusion or inclusion can be performed based on electrical information, imaging data (e.g., from patient geometry data 302) or both. Exclusion/ Inclusion function 404 can be automatic, based on evaluation of the electrical and/or imaging data, or it can be manual or semiautomatic. Each area (if any) identified for exclusion or inclusion can be co-registered with mapping data 208, such that the identified areas are not utilized, or are utilized, as the case may be as part of the calculations for assessing heart function. Alternatively, Exclusion/Inclusion function 404 can be utilized to remove or include results.

Synchrony calculator 406 can be programmed to compute one or more indications of synchrony (e.g., in the form of an index) that provides an assessment of heart function as heart function data. For instance, synchrony calculator 188 can be programmed to perform one or more calculations such as computing a heart global synchrony index (GSI), an intraventricular conduction index (ICI), a segmental synchrony index (SSI), and/or a late activation index relating to heart function data 412. Synchrony calculator 406 can further be configured to compute one or more quantitative indications of synchrony based on heart conduction data 412.

Optimization component 408 can be programmed to determine or help determine one or more ablation delivery locations in the patient's heart 16. This may involve positioning one or more stimulation and/or sensing electrodes at test sites and evaluating the synchrony determined by synchrony calculator 406, or by analyzing the electrophysiological results provided by PVM 400/450. Ablation electrode(s) 514 can be positioned at the location(s) indicated by optimization component 408 based on such an evaluation.

Additionally or alternatively, optimization component 408 can be utilized to determine or help determine one or more ablation therapy parameters, such as recommended durations of ablation or the power levels of ablation that should be delivered by ablation system 500 to the patient's heart 16. Those skilled in the art will understand appreciate various approaches that can be utilized to vary the location and/or other ablation therapy parameters to achieve a desired therapeutic ablation result.

Heart function data 412 can be utilized to present an indication of heart function on display 414, which can be configured to display text and/or two- or three-dimensional graphics. For instance, the indication of heart function for each set of parameters can be provided as a graphical element that is superimposed onto a cardiac map visualized on display 414 or another display. It is to be understood and appreciated that the determination of the heart function data 412 can be performed in real time or near-real time such that the representation of the heart function on the cardiac map can provide real time guidance and information to facilitate the location and other parameters of the ablation therapy that is being provided to the patient. The ablation therapy parameters can also be provided on display 412 or another display such as display 520.

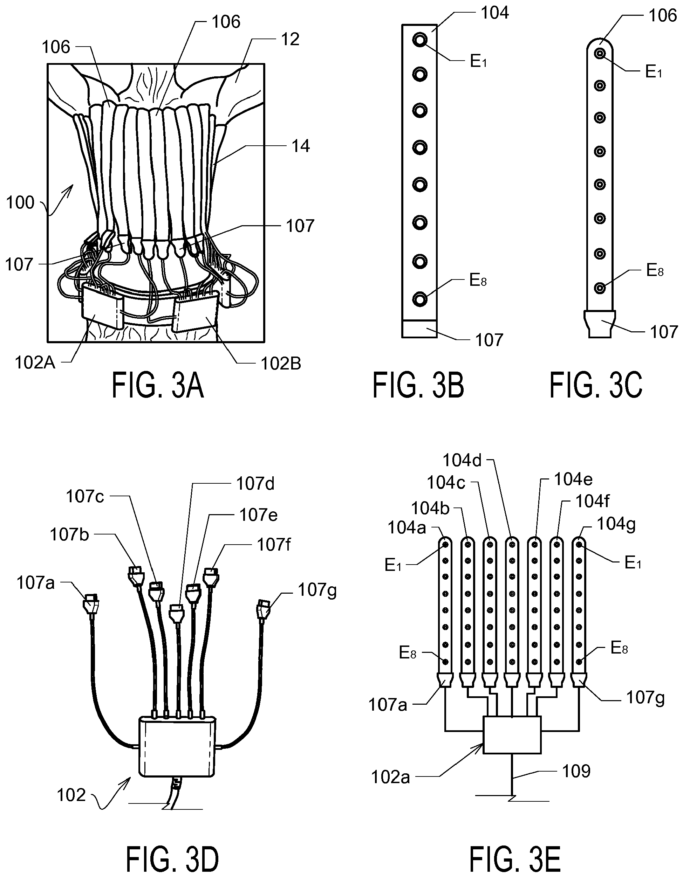

Figs. 3A through 3E show various devices and components associated with one embodiment of MES or sensor array 100.

FIG 3A shows a front view of patient 12 having strips of electrodes affixed to flat patient cables 106, where flat patient cables 106 are attached or adhered to patient's torso 14, for example by means of a biocompatible adhesive disposed on the lower surfaces of cables 106, where the adhesive is configured to permit easy removal of cables 106 from patient's torso 14 after the electrophysiological mapping procedure has been completed. In one embodiment, flat patient cables 106 (or disposable electrode strips 104 - see Fig. 3B) comprise 8 electrodes E₁ through E₈ each, and six flat patient cables 106 or disposable electrode strips 104 attached to each ECG mapping cable 102 by means of mapping cable electrode connectors 107.

Fig. 3B shows one embodiment of a disposable electrode strip 104, which comprises 8 electrodes E₁ through Es, and also comprises on its lower surface a biocompatible adhesive that permits easy removal of electrode strip 104 from patient's torso 14 after the electrophysiological mapping procedure has been completed. Disposable electrode strip 104 may also comprise mapping cable electrode connectors 107, or electrical connections may be established directly to each of electrodes E₁ through E₈ by means of separate electrical connections.

Fig. 3C shows one embodiment of a flat patient cable 106, which comprises 8 electrodes E₁ through E₈, and also comprises on its lower surface a biocompatible adhesive that permits easy removal of electrode strip 106 from patient's torso 14 after the electrophysiological mapping procedure has been completed. Flat patient cable 106 may also comprise mapping cable electrode connectors 107, or electrical connections may be established directly to each of electrodes E₁ through E₈ by means of separate electrical connections.

Fig. 3D shows one embodiment of an ECG mapping cable 102, which is configured to permit operable electrical connection thereto of seven separate disposable electrode strips 104 or seven flat patient cables 106 via mapping cable electrode connectors 107a through 107g. Mapping cable data acquisition module connectors 109 of ECG mapping cable 102 are configured for attachment to corresponding electrical connectors disposed in data acquisition device 210.

Fig. 3E shows one embodiment of an ECG mapping cable 102 operably connected to seven separate disposable electrode strips 104 or seven flat patient cables 106, each containing 8 electrodes E₁ through E₈ via mapping cable electrode connectors 107a through 107g.

Referring now to Figs. 3A through 3E, it will be seen that measurements and sensing of a patient's body surface potentials may be carried out using various electrode configurations. In one embodiment, patient cables 107 with 8 channels each are employed for such measurements and sensing. Patient cables 107 may be attached with snaps to disposable electrode strips with 8 electrodes each see Figs. 3B and 3C). In one embodiment, up to 7 patient cables may be connected to each of 4 ECG mapping cables 102. Such a configuration provides up to 224 electrodes E. See, for example, Fig. 32A, which does not show 2 additional mapping cables 102 and corresponding patient cables 107 and flat patient cables 106 or disposable electrode strips 104and , which are applied to patient's torso 14 for multichannel ECG recording.

In addition, and by way of non-limiting illustrative example, additional electrodes and electrode cables may also be affixed to patient's torso 14 to record, for example, surface electrode channels N, R, L, F, V1, V2, V3, V4, V5 and V6, as is well known in the art, and which are used to produce standard 12-lead ECG surface electrode recordings (namely, 6 extremity leads and 6 precordial leads representing extremity lead I (from the right to the left arm), lead II (from the right arm to the left leg), lead III (from the left arm to the left leg), AVL (points to the left arm), AVR (points to the right arm), and AVF (points to the feet) and precordial, or chest leads, V1,V2,V3,V4,V5 and V6 to observe the depolarization wave in the frontal plane.

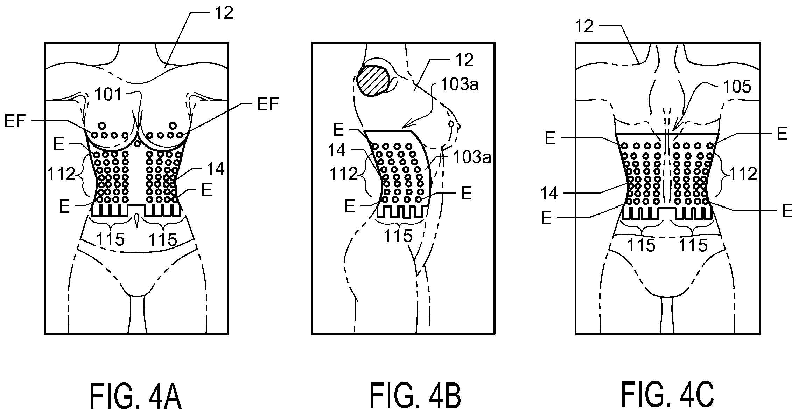

Referring now to Figs. 4A though 4C, there are shown some embodiments of customizable patches 101, 103 and 105 that can be used to simplify and speed up accurate placement of ECG electrodes on patient's torso 14. Some embodiments of patches 101, 103 and 105 permit body surface ECG signal acquisition to be performed quickly and easily, and also to be combined quickly and easily with non-invasive mapping and navigation tools, cardioversion techniques, and invasive and non-invasive ablation methods. As will become apparent to those skilled in the art upon having read and understood the present specification and claims, patches 101, 103 105 increase the efficiency and reduce the time required to carry out electrophysiological studies and mapping, increase patient comfort, are easily adaptable to changes in patient morphology, reduce ECG sensor noise, and may be combined easily with at least some other medical sensing and treatment procedures. The application to Cailler further describes and discloses details concerning patches 101, 103 and 105.

Continuing to refer to Figs. 4A through 4C, there are shown, respectively, embodiments of customizable electrophysiological mapping sensor front patch 101, one embodiment of customizable electrophysiological mapping sensor side patch 103a, and one embodiment of customizable electrophysiological mapping sensor back patch 105 mounted on, adhered or otherwise affixed to torso 14 of patient 12. As shown in Figs. 4A through 4C, each of patches 101, 103a and 105 comprises a plurality of sensing electrodes E, which in one embodiment are unipolar electrodes integrated into a fabric or other flexible material(s) from which each of patches 101, 103a and 105 is formed (more about which is said below). Rather than attach a plurality of individual electrode strips 104 or patient cables 106 to patient's torso 14, it will be seen that patches 101, 103a (and 103b - not shown in Figs. 4A through 4C, but configured similarly to patch 103a to sense ECG signals on the side opposite patch 103a of the patient's torso 14), and 105 are considerably less labor intensive and time consuming to place on patient 10. In Figs. 4A through 4C, proximal electrical connections 115 are configured for attachment to corresponding ECG mapping cable connectors 107, or to any other suitable electrical connector configured to convey electrical signals generated by sensing electrodes E to data acquisition device 210.

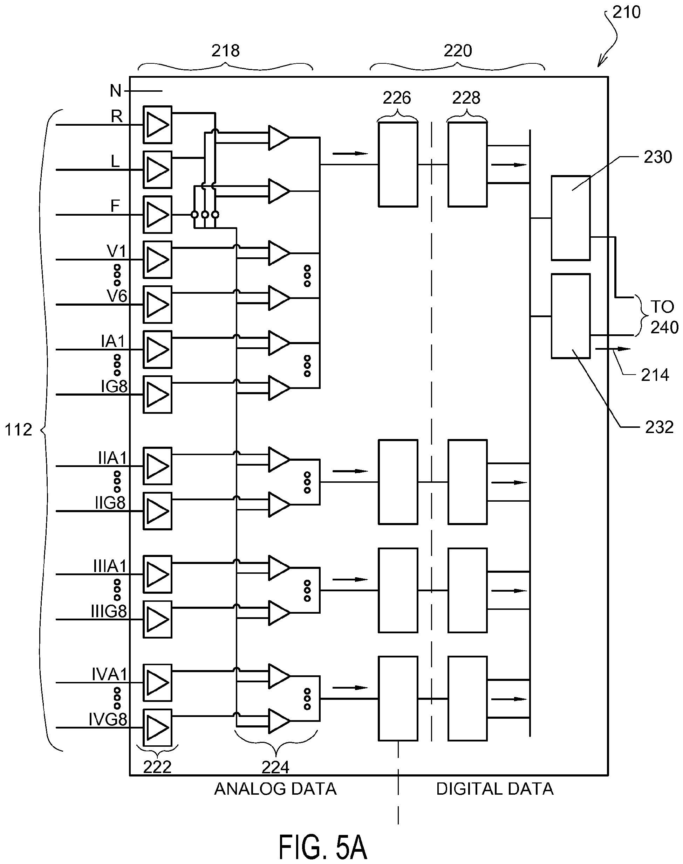

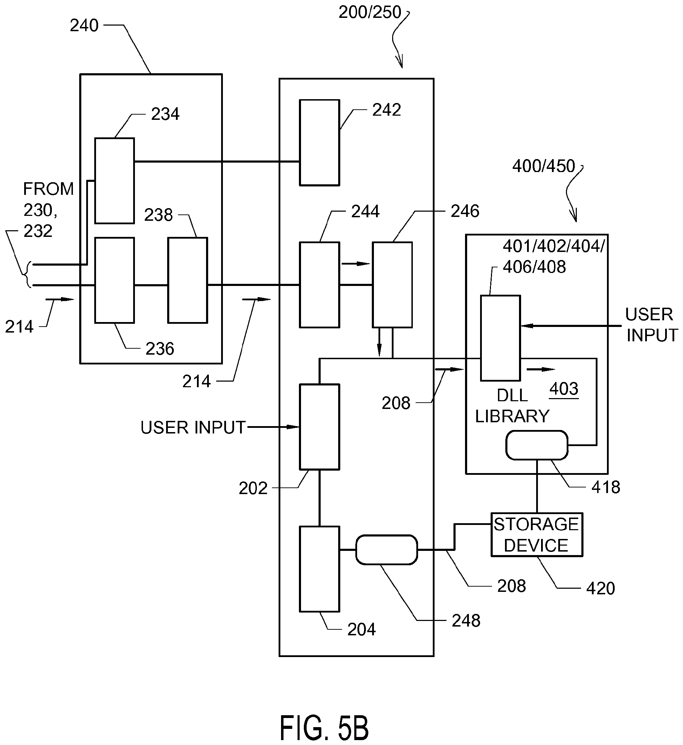

Figs. 5A and 5B show one embodiment of selected portions of system 10, including measurement system or data acquisition device 210, interface cable box 250 disposed between data acquisition device 210 and MMU 200/250, and PVM 400/450. Data acquisition device 210 is configured to interface with MMU 200 through interface cable box 215. For noninvasive cardiac mapping, and according to the various embodiments described and disclosed herein, computed tomography or magnetic resonance imaging and positional data of the patient are required as inputs to MMU 200/ 250, along with amplified, filtered and digitized ECG data provided by data acquisition device 210 through interface cable box 240. As described above, PVM 400 is configured to receive and process the tomographic images and data processed and generated by MMU 200.

Fig. 5A illustrates one embodiment of data acquisition device 210, which is configured to amplify, filter and convert into a digital format the analog signals 112 sensed by the various surface electrodes attached to the patient's torso 14 and provided by MES/sensor array 100, and to send such digital signals to the MMU 200/250 via interface cable box 240. In turn, MMU 200/250 is configured to interface with PVM 400/450, which generates and displays noninvasive cardiac mapping results.

As further shown in Fig. 5A, and in one embodiment, each of the analog electrode signals 112 acquired from the patient's torso 14 (except that of the neutral electrode) is input into data acquisition device 210 through one of the repeaters/matching amplifiers 222. Analog signals 112 corresponding to the ECG limb electrodes R, L and F are then routed into two of differential amplifiers 224 to produce ECG lead I and ECG lead II signals, respectively. Further, each of the 224 analog signals of the ECG mapping cables and each of the analog signals of the precordial electrodes are led through separate differential amplifiers 224 (having, for example, a common mode rejection ratio > 105 dB @ 50 Hz) which employs a reference signal produced from the other electrode signals). Through the neutral electrode N, a signal is applied to the patient's torso 14 body to counteract or diminish common mode noise in the acquired ECG signals.

Once amplified, the collected analog ECG signals are converted into digital signals with four 24-bit analog-to-digital converters 226, each being configured to convert, by way of non-limiting example, up to 64 channels of analog input signals 112 into digital signals at a sampling rate of, for example, 1 kHz (although other sample rates are contemplated). The digital signals are then processed by four micro-controllers, controllers, processors, microprocessors and/or CPUs 228, which send the measurement data or digital signals 214 organized into a suitable digital format to interface cable box 240 using, for example, an RS-232 serial communication standard for transmission of data. To protect the electrical circuits of data acquisition device 210 and those of the electrodes operably connected thereto from harmful currents, DC-DC converters 230 and 234 in combination with galvanic isolation modules 232 and 236 may be employed on both ends of interface cable box 240 to operably connect data acquisition device 210 to interface cable box 250.

MMU 200/250 receives the digital signals 214 provided by the data acquisition device 210 through the interface cable box 240 through, by way of non-limiting example, an integrated RS-232-to-USB interface module, a universal serial bus (USB) cable, or a flash drive. MMU 200/250 collects the data provided by data acquisition device 210 through USB driver 244 and organizes the incoming binary ECG data into packets using a computer algorithm stored in a suitable non-transitory computer readable medium of MMU 200/250 configured, by way of non-limiting example, as a dynamic-link library (DLL) 246. The data packets are then processed in DLL 246 in conjunction with suitable operator interface algorithms loaded in operator interface module 202, and may then be displayed on a graphical output device 216 of MMU 200/250. The data may be further processed in MMU 200/250 using a suitable data review algorithm loaded in data review module 204, which allows a user to select desired time portions of ECG data included in measurement data 214, and to store such portions in a suitable ECG data format. The selected and formatted data (e.g., mapping data 208) may be written or transferred to a suitable memory or storage device (e.g., RAM, a USB flash drive, etc.) via a USB driver 248 or other suitable means (e.g., Ethernet or network connection). Alternatively, MMU 200/250 is configured to transfer mapping data or data packets 208 directly to PVM 400/450 by means of one or more network interfaces that use, for example, the Transmission Control Protocol and the Internet Protocol (TCP/IP).

A second DLL 403 may be included in PVM 400/450, and employs computer algorithms configured to receive mapping data 208, and to process and analyze the mapping data 208.

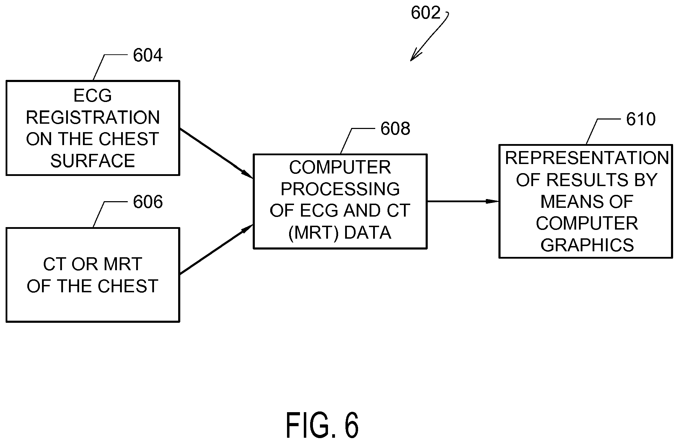

Fig. 6 illustrates a general schematic view of portions of the methods described and disclosed herein. The method 602 includes: (1) Step 604 (registration of surface electrodes attached to the patient's torso and configured to acquire ECG therefrom); (2) Step 606 (acquisition of CT (computed tomography) data and/or MRT magnetic resonance tomography)/MRI (magnetic resonance imaging) data and ECG electrode position data from the patient's torso); (3) Step 608 (data processing of surface ECG data and of CT data and/or MRT/MRI data) using computing techniques), and (4) Step 610 (visual representation(s) of the obtained electrophysiological information by means of computer graphics processing).

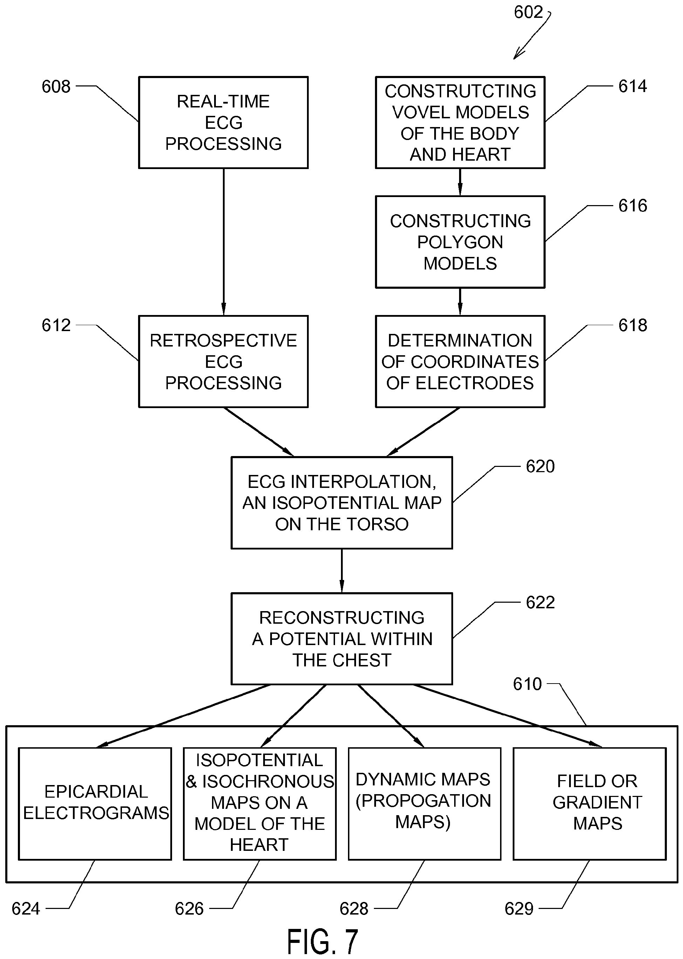

Fig. 7 illustrates a further schematic view of one embodiment of the main stages of computer processing of the surface electrograms signals acquired from the patient's body 12 or torso 14. Step 608 comprises real-time or near-real-time processing of ECG signals, which may be combined with multi-channel ECG electrode registration from the patient's torso generated using CT and/or MRT/MRI data. Step 612 comprises retrospective processing of ECG signals. Step 614 comprises processing of ECG signals, and includes constructing voxel models of the torso, heart and its compartments, also using, by way of non-limiting example, CT or MRT/MRI derived data. Step 614 comprises constructing voxel models of the torso, heart and its compartments. Step 616 comprises constructing polygonal or other surfaces or volume models of the torso, heart and its compartments, and may be carried out, by way of example, using boundary element or finite element modelling (FEM) techniques. Step 618 comprises manual and/ or (semi-) automatic determination of the spatial coordinates of surface electrodes on the torso surface, also using, for example, CT and/or MRT/MRI derived data. At step 620, surface interpolation of values of surface mapping ECG signals at each discrete moment in time and construction of isopotential maps on the torso surface are performed. Step 622 comprises computational reconstruction of the heart electrical field potential on the heart's epicardial and/or endocardial surfaces. In the last steps 610, reconstructing epicardial and/or endocardial electrograms occurs (Step 624), epicardial and/or endocardial isopotential isochronous maps are constructed by means of computer graphical processing and computer graphics on a realistic computer model of the heart (step 626), and/or visualizing the dynamics of electrophysiological processes of the epicardium, myocardium and/or endocardium in animation mode (propagation mapping) are performed (step 628), respectively.

Each of the foregoing steps is described in detail in the aforementioned '547 '461 patent and '639 patents. Some of the above steps are described in further detail in: U.S. Patent No. 7,016,719 to Rudy et al. entitled "System and method for noninvasive electrocardiographic imaging (ECGI) using generalized minimum residual (GMRES)" (hereafter "the '719 patent").

In addition, certain aspects of the steps described and disclosed herein are described in at least some of the following publications and portions of publications, namely:
Revishvili, et al., "Electrophysiological Diagnostics and Interventional Treatment of Complex Cardiac Arrhythmias with Use of the System of Three-Dimensional Electro-Anatomical Mapping," pp. 32-37 (2003);
Titomir, et al., "Noninvasive Electrocardiotopography," pp. 97-111 (2003);
Shakin, "Computational Electrocardiography," Nauka, pp. 64-65 (1981);
Golnik, et al., "Construction and Application of Preprocessor for Generation, Performance Control, and Optimization of Triangulation Grids of Contact Systems," pp. 1-25 (2004);
Titomir, et al., "Mathematical Modeling of the Cardiac Bioelectric Generator," Nauka, pp. 329-331 (1999);
Lacroute, "Fast Volume Rendering Using a Shear-Warp Factorization of the Viewing Transformation," Computer Systems Laboratory, Depts. of Electrical Engineering and Computer Science, Stanford University, pp. 29-43 (1995);
Lorensen, et al., "Marching Cubes: A High Resolution 3D Surface Construction Algorithm," Computer Graphics, vol. 21, No. 4, pp. 163-169 (1987);
Saad, "Iterative Methods for Sparse Linear Systems," Second Edition with Corrections, pp. 2-21, 157-172 (Jul. 2000);
Rudy, et al., "The Inverse Problem in Electrocardiography: Solutions in Terms of Epicardial Potentials," Crit Rev Biomed Eng., pp. 215-268 (1988); Abstract.
Berger, et al., "Single-Beat Noninvasive Imaging of Cardiac Electrophysiology of Ventricular Pre-Excitation," Journal of the American College of Cardiology, pp. 2045-2052 (2006).
Lo, "Volume Discretization into Tetrahedra--II. 3D Triangulation by Advancing Front Approach," Computers & Structures, vol. 39, Issue 5, pp. 501-511(1991);
Rassineux, "3D Mesh Adaption. Optimization of Tetrahedral Meshes by Advancing Front Technique," Computer Methods in Applied Mechanics and Engineering 141, pp. 335-354 (1997);
Yoshida, "Applications of Fast Multipole Method to Boundary Integral Equation Method," Dept. of Global Environment Eng., Kyoto Univ., Japan, pp. 84-86 (Mar. 2001);
Kazhdan, et al., "Poisson Surface Reconstruction," Eurographics Symposium on Geometry Processing (2006);
Schilling, et al., "Endocardial Mapping of Atrial Fibrillation in the Human Right Atrium Using a Non-contact Catheter," European Heart Journal, pp. 550-564 (2000);
Ramanathan, et al., "Noninvasive Electrocardiographic Imaging for Cardiac Electrophysiology and Arrhythmia," Nature Medicine, pp. 1-7 (2004);
MacLeod, et al., "Recent Progress in Inverse Problems in Electrocardiology," Nora Eccles Harrison Cardiovascular Research and Training Institute, University of Utah, pp. 1-20, 1998;

Continuing to refer to Fig. 7, in Step 608 real-time or near-real-time processing of ECG signals is carried out, which may be combined with multi-channel ECG electrode registration from the patient's torso generated using CT and/or MRT/MRI data. According to one embodiment, in the course of real-time or near-real-time ECG mapping, surface ECG signals that have been acquired from the patient's torso 14 may be displayed on a computer monitor or display to a user and/or health care provider. The user controls the quality of ECG signals from each of the leads; if necessary, a programmed suppression of power-line, muscle noise and of isoline- or DC-drift is applied. Automatic control and editing of the quality of acquired ECG signals may also be carried out based on spectral and mutual-correlation analyses of ECG signals. Results obtained in Step 608 are digitized and filtered values of the ECG signals, and may include, by way of example, signals from 224 or 240 unipolar leads located on the patient's torso and 12 standard leads with the duration. In one embodiment, ECG signals are acquired from the patient for up to 1, 2, 3, 4 or 5 minutes.

Still referring to Fig. 7, in Step 612 "retrospective processing" of ECG signals occurs. In one embodiment, the user and/or health care provider looks through the acquired ECG signals and selects one or several cardiocycles for subsequent processing. Further, a reduction of ECG to a united isoline may be implemented: to this end, in one of the ECG signals the user selects a time interval tau, within which an ECG-signal coincides with an isoline (for example, this interval may belong to a cardiac signal segment PQ). Correction of ECG signals is implemented according to the formula: U₀(t)=U(t)-u₀, where U₀(t) is the selected and corrected ECG-signal, U(t) is an initial ECG signal, and uo is an averaged value of the initial ECG signal within a time interval tau. Afterwards, the user selects a time interval of interest in the cardiac cycle for subsequent calculations.

In Step 614 of Fig. 7, voxel models of the torso and heart are constructed using a voxel graphics editor. Using the aforementioned CT or MRT/MRI or other electrode, sensor or transducer spatial position/location data of the patient's torso 14 and heart 16, a voxel rendering of anatomical structures of the torso 14 is provided. To this end, and in one embodiment, a "shear-warp factorization" of the viewing transformation algorithm, which belongs to a group of scanline-order volume rendering methods, may be used. In one embodiment, the voxel rendering method applied comprises three main steps. In a first step, volume data are transformed by a shear matrix in the corresponding object space, each parallel slice of volume data after transformation being passed through a filter configured to for diminish distortions in the volume data. In a second step, an intermediate 2D image within the same shear space is formed from a combined set of filtered and sheared slices using direct-order superposition. In a third step, the intermediate 2D image obtained is transformed into a normal image space using a shear matrix and is then passed through a filter to form the final image. See, for example, Philippe Lacroute , "Fast Volume Rendering Using a Shear-Warp Factorization of the Viewing Transformation," Ph.D. dissertation, Technical Report CSL-TR-95-678, Stanford University, 1995.

In Step 616 of Fig. 7, polygonal surfaces (or triangulation grids) and/or tetrahedral models and/or any other kind of suitable geometric models of the torso and heart may be constructed on the basis of the voxel models calculated and provided in Step 614. In one embodiment, and based on the obtained voxel models, polygonal surfaces consisting of united plane triangles are automatically constructed. The Initial data employed in such a construction are representative of a three-dimensional scalar field of densities provided in in a voxel presentation or format (i.e., a three-dimensional right-angled grid, in whose nodes values of the conditional densities of torso tissues are provided). Constructing triangulation grids of the torso and heart is accomplished by constructing polygonal surfaces, which may be repeated surfaces of the structures provided by the three-dimensional scalar density field. Other types of modelling techniques may be used in Step 618, such as finite element models.

In one embodiment, a procedure for constructing polygonal surfaces includes the following steps: filtering initial voxel models to reduce or diminish undesired noise; constructing a triangular surface on the basis of a "marching cubes" algorithm and "exhaustion method" (also known in the literature as an "advancing front" algorithm); smoothing the resulting grid of surface values (i.e., constructing a polygonal surface close to the initially-derived polygonal surface but differing from it by having lower values of angles between the normal vectors of adjacent triangles; and rarefying and quality-improving the smoothed grid of surface values of the polygonal grid (i.e., constructing a polygonal surface with a lower number of larger triangles, which are close to equilateral triangles). A "marching cubes" algorithm permits the construction of a polygonal representation of isosurfaces given by a three-dimensional scalar field of densities. For further details regarding such steps, see, for example, the '547'461, '639 and '719 patents. See also, for example: (1) W. Lorensen, H. Cline, "Marching Cubes: A High Resolution 3D Surface Construction Algorithm," Computer Graphics, 21(4): 163-169, July 1987). (2) Lo, S. H., "Volume Discretization into Tetrahedra, II. 3D Triangulation by Advancing Front Approach," Computers and Structures, Pergamon, Vol. 39, No. 5, p.p. 501-511, 1991; (3) Rassineux, A. "Generation and Optimization of Tetrahedral Meshes by Advancing Front Technique//International Journal for Numerical Methods in Engineering," Wiley, Vol. 41, p.p. 651-674, 1998; (4) Gol'nik, E. R., Vdovichenko , A. A., Uspekhov, A. A., "Construction and Application of a Preprocessor of Generation, Quality Control, and Optimization of Triangulation Grids of Contact Systems," Information Technologies, 2004, No. 4, p. 2-10.

In Step 618 of Fig. 7, and according to one embodiment, automatic determination of the spatial three-dimensional coordinates of the ECG electrodes attached to patient torso is carried out using the previously acquired CT or MRT/MRI data of the patient's torso. Initial tomography data are digitally filtered using a predetermined density threshold such that only those tomography data are retained that correspond to the density levels of the various surface ECG electrodes. On the basis of a new voxel model computed using the filtered tomography data, a multi-electrode triangulation grid is constructed using the "marching cubes" method. For each electrode location in the triangulation grid, the coordinates of its geometrical center are calculated as an arithmetical mean of the coordinates of its corresponding nodes. For each region, the Euclidean distance from its geometrical center to the nearest point of the surface of the torso is calculated. Regions with the Euclidean distances exceeding a predetermined threshold are rejected. Geometric centers of the remaining regions are assumed to be the Cartesian coordinates of electrodes in three-dimensional space. In accordance with such an ECG electrode spatial positioning and determination scheme, the spatial coordinates are calculated and assigned to each ECG electrode. During this step, the user and/or health care provider may have the option to correct the positions each of electrode in an interactive mode.

In Step 620 of Fig. 7, an isopotential map of the torso surface 18 is constructed. In one embodiment, construction of isopotential maps may be carried out by surface interpolation of values of ECG signals at each discrete moment in time using radial basis functions. The electric field potential on the surface of the torso may be represented in the form of decomposition according to a system of radial basis functions, as described in the '660 patent. To compute the potential at each point of the torso surface, a bilinear interpolation of values in vertices of a grid triangle may be applied.

Such a method may include noninvasive reconstruction of the heart's electrical field potential at internal points of the torso based on measured values of the electric field potential on the torso surface by numerically solving the inverse problem of electrocardiography for an electrically homogenous model of the torso by a direct boundary element method on the basis of an iteration algorithm, as also described in the '660 patent. Solution of the inverse problem of electrocardiography may comprise a harmonic continuation of the potential u(x) from the surface. See, for example, Brebbia, C., Telles, J., and Wrobel, L., "Boundary element techniques," Moscow, Mir (1987). The external surface of the heart and surfaces bounding the torso may be approximated by a boundary-element grid, i.e., a polygonal surface comprising plane triangles, which are split into boundary elements. The potential u(s) and its normal derivative q(s) may be represented in the form of decomposition according to a system of linearly independent finite basis functions, where coefficients of decomposition uᵢ and qᵢ are values of the potential u(s) and its normal derivative q(s) in nodes of a boundary-element grid. As a result, a number of vectors are formed. The direct boundary element method may employ Green's third (main) formula, which connects values of the potential and its normal derivative at boundary surfaces with values of the potential within the computational domain . Use of Green's third formula for points laying on surfaces yields a system of Fredholm integral equations, which may be written in the form of a system of two matrix-vector equations with two unknown vectors uₕ and qₕ after boundary-element discretization of functions u(s) and q(s). An iteration algorithm is then employed, which may involve applying the Morozov principle and the Tikhonov regularization method. In one embodiment, the total number of triangle elements in a grid for the torso and heart is about 2252. To model the standard electric field of the heart, a quadruple source can be placed in a geometric center of the heart. The construction of isopotential maps is thus carried out by surface interpolation of values of ECG signals at each discrete moment in time with using radial basis functions. Further details concerning Step 620 are set forth in the '547 '461, '639 and '719 patents, as well as in some of the publications referenced herein.

In Step 622 of Fig. 7, the electric field of the heart's surface is computed, and in one embodiment an algorithm and method similar to that disclosed in the '719 patent is employed, which involves application of a generalized minimum residual (GMRES) algorithm. The parameters of the GMRES algorithm, of a model for the torso 14 and heart 16, and of a standard electric field may be the same as those described above in connection with the '719 patent, and are also discussed in detail in the 547, '461 and '639 patents (as well as in some of the publication referenced herein). See also, Saad, Y. "Iterative Methods for Sparse Linear Systems," (2nd ed.), SIAM, Philadelphia (2003).

Continuing to refer to Fig. 7, in the last steps 610 shown therein, reconstructing epicardial and/or endocardial electrograms occurs (Step 624), epicardial and/or endocardial isopotential isochronous maps are constructed by means of computer graphical processing and computer graphics on a realistic computer model of the heart (step 626), and/or visualizing the dynamics of electrophysiological processes of the epicardium, myocardium and/or endocardium in animation mode (propagation mapping) are performed (step 628), respectively, using the methods and techniques described above and in the various patent and literature publication referenced herein.

Using the foregoing techniques and methods, it will now be seen that various types of visual representations of the electrical activity of the patient's heart can be provided by the above-described non-invasive external electrophysiological mapping system or EMS 10. In one embodiment, EMS 10 comprises: (a) a plurality of surface electrical sensing electrodes E configured to acquire surface electrocardiogram (ECG) signals from at least portions of patient's torso 14; (b) data acquisition device 210 operably connected to the surface electrical sensing electrodes E and configured to condition the ECG signals provided thereby; (c) at least one non-transitory computer readable medium storing instructions executable by at least one processor to perform a method for receiving and processing the ECG signals in, for example, MMU 200 and first computer or computer workstation 250, PVM 400 and second computer or computer workstation 450, and/or in another suitable computing platform, whether local or remote, thereby to provide on a display or monitor a real-time or near-real-time voxel-model-derived visual representation or image of at least a portion of the patient's heart during an electrophysiological mapping procedure. The visual representations or images of the electrical activity of the patient's heart, endocardium, epicardium, or myocardium provided by EMS 10 can include epicardial or endocardial electrograms of a patient's heart, isopotential, isochronous maps of a model of a patient's heart, and/or dynamic or electrical wavefront propagation maps of a patient's heart, or other types of visualizations or images that can be generated by EMS 10. As described in further detail below, such visual representations of the electrical activity of a patient's heart may be provided in combination with a cardiac ablation procedure carried out using an ablation system 500, which is configured to operate in conjunction with EMS 10.

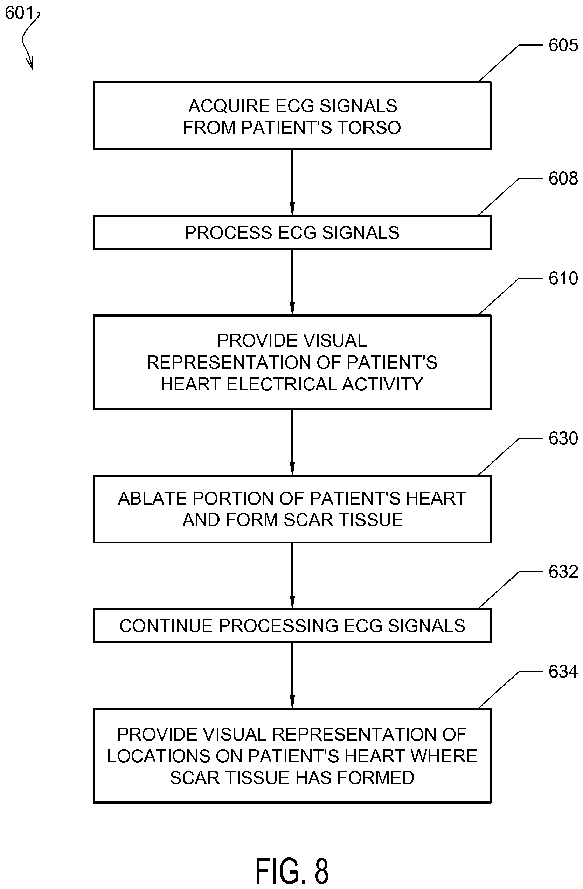

Referring now to Fig. 8, there is shown one embodiment of a combined method 601 of electrophysiological mapping and ablation of a patient's heart 16, where a visual representation of at least one location where scar tissue has been or is being formed in or on a patient's heart 16 during the combined method is shown to a user on a monitor or display. At step 605, during the combined procedure ECG signals are acquired from surface 18 of patient's torso 14 using MES/sensor array 100. At step 608, the ECG signals are processed in MMU 200/250 and/or PVM 400/450 using the various data processing techniques and methods techniques described above. At step 610, one or more visual representations or images of electrical activity occurring over at least a portion of the patient's heart 16 during the combined procedure are provided in real-time or near-real-time on a monitor or display to the user. At step 630, a portion of the patient's heart 16 is ablated by an ablation device or system 500, and forms scar tissue on or in patient's heart 16. At step 632, and after scar tissue has been formed on or in patient's heart 16 in step 630, ECG signals continue to be acquired from patient's torso 14 using MES/sensor array 100, and continue to be processed in MMU 200/250 and/or PVM 400/450. At step, 634, in real-time or near-real-time, one or more visual representations or images 420 of one or more locations on or in the patient's heart where the scar tissue has been formed or is being formed by the ablation device or system 500 are provided to the user on the monitor or display to the user.

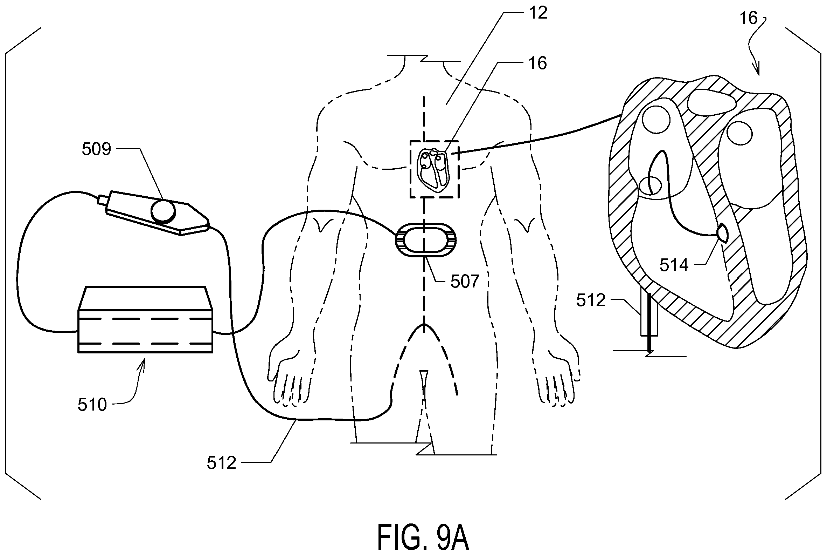

The combined method 601 shown in Fig. 8, and also in Figs. 9A through 10(f), is carried out using EMS 10 comprising a plurality of surface electrical sensing electrodes E included in MES 100, which are configured to acquire ECG signals from at least portions of a patient's torso, a data acquisition device or measurement system 210 operably connected to the surface electrical sensing electrodes and configured to condition the ECG signals provided thereby, at least one non-transitory computer readable medium (e.g., computer 250 or 450) storing instructions executable by at least one processor to perform a method for receiving and processing the ECG signals to provide on a display or monitor a real-time or near-real-time voxel-model-derived visual representation or image of at least a portion of the patient's heart 16 during the combined electrophysiological mapping and cardiac ablation procedure carried out on the patient 12.

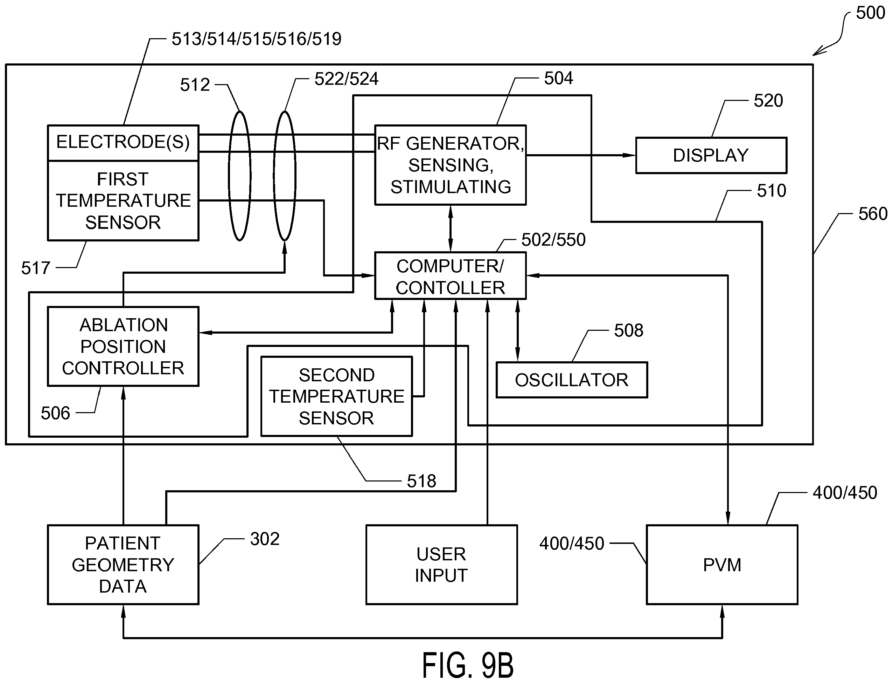

In one invasive embodiment, the combined method 610 shown in Fig. 8 may further be carried out using a cardiac ablation system 500 comprising an invasive cardiac ablation system, which comprises an ablation catheter 512 configured for insertion inside the heart 16 of patient 12. Ablation catheter 512 comprises a distal end comprising a tissue ablation device (e.g., an RF ablation electrode, a laser-beam-emitting ablation device, a chemical ablation device, a cryogenic device, a radiation device, or a particle-beam-emitting device) configured to controllably form scar tissue on the patient's endocardium during the combined electrophysiological mapping and cardiac ablation procedure. See, for example, Figs. 9A and 9B.

In another non-invasive embodiment, the combined method 610 shown in Figs. 8, 9A and may further be carried out using a cardiac ablation system 500 comprising an external non-invasive cardiac ablation system, which comprises at least one external directionally controllable and focusable source of ablation energy 522 (e.g., a HIFU transducer or particle beam generator), ablation energy source being configured to controllably form scar tissue on the patient's endocardium during the combined electrophysiological mapping and cardiac ablation procedure. See, for example, Fig. 9C.

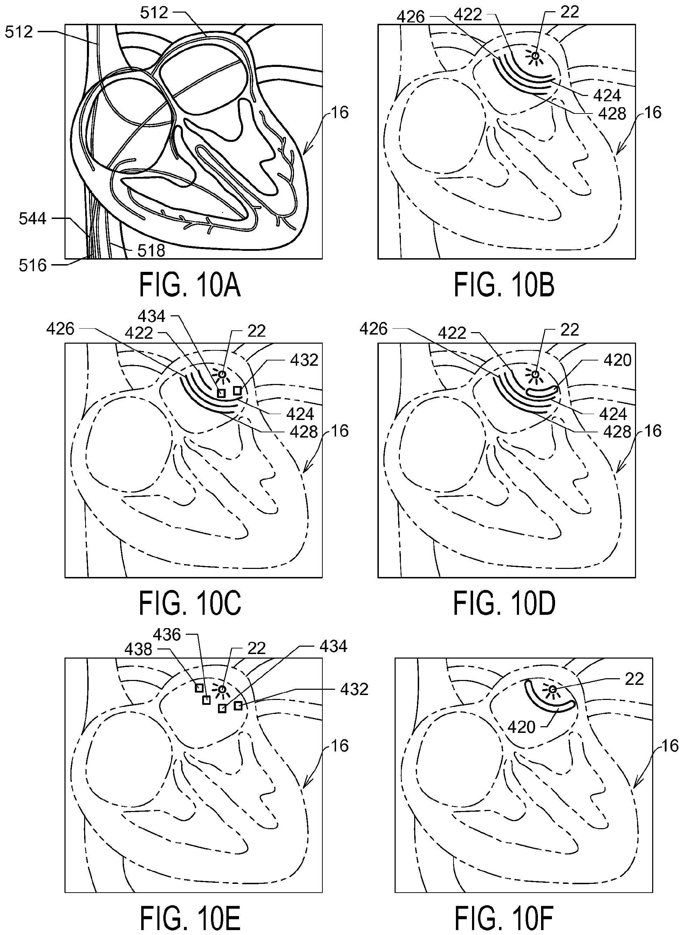

Whether invasive or non-invasive embodiments of cardiac ablation system 500 are employed, the EMS is further programmed and configured to process the ECG signals during the combined electrophysiological mapping and cardiac ablation procedure 601 to produce on a display or monitor one or more real-time or near-real-time voxel-model-derived visual representations or images 420 of one or more locations on the patient's heart 16 where at least one scar has been created by the ablation device or ablation energy source during the combined electrophysiological mapping and cardiac ablation procedure. See Figs. 10(a) through 10(f).

The visual representations or images of scarring locations may be based upon one or more of a velocity field or gradient, an amplitude field or gradient, an electrical conductivity field or gradient, or an electrical impedance field or gradient calculated by PVM 400 for at least a portion of the patient's heart 16.
wherein the cardiac ablation system further comprises an electrical stimulation electrode located near or at the distal end of the catheter, the electrical stimulation electrode being configured to stimulate electrically intracardiac tissue of the patient to produce an evoked response therein, the EMS being configured to detect ECG signals corresponding to the evoked response and process such signals to provide or refine the visual representation of the intracardiac location where scarring created by the ablation device has occurred.

In an invasive embodiment of a system for combined electrophysiological mapping and ablation of a patient's heart 16, and referring to Figs. 9A, 9B and 10(a) through 10(f), a location of the distal end of catheter 512 in patient's heart 16 may be provided as a visual representation or image to a user on the basis of a point of origin of an evoked response in heart 16 that is calculated by PVM 400 in response to a stimulation pulse being provided to heart 16 by a stimulation or pacing electrode that is incorporated into ablation catheter 512, or alternatively that is provided using a separate pacing catheter. In an invasive embodiment of ablation system 500, ablation system 500 and controller 502 may be configured together to control a power level or duty cycle of the ablation delivered by the ablation device to the patient's heart 16, where the power level or duty cycle delivered by ablation system 500 is based on an amount, degree or extent of scarring of the patient's heart determined at least partially to have occurred by EMS 10. An amount of time ablation is delivered by ablation system 500 to the patient's heart 16 may also be calculated by EMS 10 (e.g., PVM 400 and/or ablation system 500), the amount of time being based on an amount, degree or extent of scarring of the patient's heart determined at least partially to have occurred by EMS 10. As described above, ablation catheter 512 may further comprise near or at its distal end at least one electrode, coil, sensor, transducer, magnetic source, or antenna that in combination with the EMS is configured to permit a location of the catheter's distal tip within the patient's heart to be determined and displayed on a monitor or display in real-time or near-real-time. Catheter 512 may also comprise near or at its distal end at least one electrical sensing electrode configured to sense electrical signals generated by the heart, be it an evoked response prompted by a pacing electrode or natural electrical signals originating in heart 16. The sensed electrical signals are provided thereby to EMS 10 as input signals thereto for further processing, and analysis and/or display as visual representations or images.

In a non-invasive system for combined electrophysiological mapping and ablation of a patient's heart, and referring to Figs. 9C and 10(a) through 10(f), a visual representation or image of the scarring location formed by ablation system 500 may also be based upon a velocity field or gradient, an amplitude field or gradient, an electrical conductivity field or gradient, or an electrical impedance field or gradient of at least a portion of the patient's heart, the field or gradient being calculated by the EMS. By way of example, the ablation energy source of a non-invasive cardiac ablation system may comprise a high intensity focused ultrasound (HIFU) system, a proton beam radiotherapy system, or an X-ray beam radiotherapy system.

In one embodiment, a magnetic resonance imaging (MRI) and guiding system may be included in imaging system 300 and configured in conjunction with EMS 10 to provide a three-dimensional image of at least a portion of the patient's heart 16, and to guide or help guide a location 544 of the ablation energy that is applied to the patient's heart 16 during the combined electrophysiological mapping and cardiac ablation procedure. The MRI and guiding system 300 and EMS 10 may be configured together to produce on a display or monitor to the user one or more real-time or near-real-time two- or three-dimensional visual representations or images of at least a portion of the patient's heart 16 and the locations 544 of the ablation energy applied to the patient's heart.

Alternatively, a computer tomography (CT) imaging and guiding system may be included in imaging system 300 configured in conjunction with EMS 10 to generate one or more real-time or near-real-time two- or three-dimensional visual representations or images of at least a portion of the patient's heart 16, and to guide the location 544 of the ablation energy that is applied to the patient's heart during the combined electrophysiological mapping and cardiac ablation procedure. The CT imaging and guiding system 300 and EMS 10 may be configured together to produce on a display or monitor to the user one or more real-time or near-real-time two- or three-dimensional visual representations or images of at least a portion of the patient's heart 16 and the locations 544 of the ablation energy applied to the patient's heart.

In another embodiment, an ultrasound imaging and guiding system may be included in imaging system 300 configured in conjunction with EMS 10 to generate one or more real-time or near-real-time two- or three-dimensional visual representations or images of at least a portion of the patient's heart 16, and to guide the location 544 of the ablation energy that is applied to the patient's heart during the combined electrophysiological mapping and cardiac ablation procedure. The ultrasound imaging and guiding system 300 and EMS 10 may be configured together to produce on a display or monitor to the user one or more real-time or near-real-time two- or three-dimensional visual representations or images of at least a portion of the patient's heart 16 and the locations 544 of the ablation energy that has been or is being applied to the patient's heart. Similar to some of the invasive embodiments of ablation system 500 described above, in non-invasive embodiments of ablation system 500, EMS 10 and ablation system 500 are configured to control a power level or duty cycle of, or amount of time, ablation is delivered by the non-invasive ablation device to patient's heart 16, where the power level or duty cycle is based on an amount, degree or extent of scarring of the patient's heart 16 determined at least partially to have occurred by the EMS and/or ablation system 500.

Thus, it will now be seen that in invasive and non-invasive embodiments of ablation system 500 operating in conjunction with the other components and systems of EMS 10, there are provided methods of visualizing on a monitor or display at least one location where scar tissue has been or is being formed in or on a patient's heart 16 during a combined electrophysiological mapping and ablation procedure. Such methods comprise acquiring, during the combined procedure, ECG signals from a surface of the patient's torso; processing, in a combined electrophysiological mapping and ablation system, the ECG signals; providing, on the monitor or display, a real-time or near-real-time visual representation or image of electrical activity occurring over at least a portion of the patient's heart during the combined procedure; ablating a portion of the patient's heart with an ablation device and forming scar tissue thereon or therein; continuing to process, in the combined electrophysiological mapping and ablation system, ECG signals acquired or being acquired from the surface of the patient's torso; and providing, on the monitor or display, a real-time or near-real-time visual representation or image of one or more locations on the patient's heart where scar tissue has been formed or is being formed therein or thereon by the ablation device. Such methods may further comprise using spatial position data (e.g., patient geometry data 302). The spatial position data can be generated by an imaging system 300 operably connected to or forming a portion of the combined electrophysiological mapping and ablation system, EMS 10. The spatial position data can be based upon or related to calculations carried out by one of the computers included in EMS 10 (such as computer or computer workstation 450) that are used to provide one or more visual representations or images of the one or more locations where scar tissue has been formed or is being formed. Such spatial position data may also be employed to control further positioning of non-invasive ablation device 522 with respect to patient's heart 16 such that new scar tissue is formed therein or thereon in at least one desired new scar location.

Referring now to Figs. 9A and 9B, there is shown one invasive embodiment of ablation system 500, which is configured to operate in conjunction with EMS 10. Ablation system 500 may be configured to cause ablation of a selected portion of the patient's endocardium, epicardium and/or myocardium by means of radiofrequency (RF) energy, laser energy, cryogenic techniques, radiation techniques, and/or chemical ablation techniques. In the embodiments illustrated in Figs. 9A and 9B, however, ablation system 500 is shown as an RF ablation system, although some components and modules illustrated in Fig. 9B may be used in non-RF embodiments of ablation system 500.

In Fig. 9A, ablation generator and control module 510 is operably connected to and controls ablation energy delivered by ablation electrode 514 disposed at the tip of transvenous ablation catheter 512, which is routed to the patient's heart 16 via, for example, a femoral vein. Control handle 509 may be employed by a user to control the delivery and timing of ablation energy to ablation electrode 514. Reference or ground electrode 507 is also operably connected to ablation generator and control module 510, and is generally attached to the back of patient 12. The black zone depicted around ablation electrode 514 illustrates the zone in which cardiac tissue is being ablated.

In Fig. 9B, ablation system 500 comprises ablation generator and control module 510, which in turn comprises RF generator 504, ablation controller, computer, controller and/or control circuitry 502/550, optional ablation position controller 506 (which may be included in non-invasive or invasive embodiments of ablation system 500 to control the location(s) and position(s) where ablation is to occur within or on patient's heart 16, using, for example, a non-invasive transducer 522 and transducer locater 524 as in Fig. 9C), and oscillator 508. Patient geometry data 302 are provided to ablation position controller 506, which in turn may be derived or provided by PVM 400. Computer/controller 502/550 may be operably coupled and connected to PVM 400/450, which can provide ablation control, timing, power and other instructions computed according to the degree, location and type of scar formation that PVM 400/450 has detected.

RF generator 504 is electrically coupled to ablation electrode 514. Ablation electrode 514 is preferably disposed at the distal end of catheter 512. In one embodiment, first temperature sensor 517 is located near ablation electrode 514, and is configured to sense the temperature of the cardiac tissue that is being ablated by ablation electrode 514 during the ablation procedure. Controller 502/550 is operably coupled to first temperature sensor 517 and second temperature sensor 518. Using controller 502/550, the amount of power delivered by RF generator 504 to ablation electrode 514 may be modulated or controlled according to the temperature sensed by first temperature sensor 517. Controller 502/550 may be configured so that a constant power is delivered to ablation electrode 514 is maintained, or so that a constant temperature of cardiac electrode 514 is maintained. Controller 502/550 may also be configured to detect the heating efficiency of the power delivered to the ablation electrode 514. Controller 502/550 may be a separate device or integral with the RF generator 504.

In one embodiment, oscillator 508 is used to cyclically vary the signal delivered to ablation electrode 514 at a frequency ranging, by way of example, between 350 and 500 kHz. Other ablation frequencies are contemplated, as is known in the art.

In one embodiment, optional second temperature sensor 518 may be employed, which is located remote from first temperature sensor 517 but in sensory contact with the patient's body so that variations in the body temperature of the patient during the ablation procedure may be sensed and corrected. Second temperature sensor 517 may or may not be positioned along ablation catheter 512, and may be useful for those patients whose body temperature varies during the ablation procedure. For example, it is sometimes necessary to deliver a drug, such as isoproteronol, to mimic exercise and, in turn, induce arrhythmias. Such a drug, however, can cause the body temperature to rise 1 or 2 degree Celsius.

In one embodiment, ablation system 500 may include display 520, which is configured to graphically output data indicating the degree to which the electrode contacts heart tissue (e.g. no contact, medium contact, etc.). Display 520 may also provide data regarding the power delivered, electrode temperature or heating efficiency over time. Alternatively or in addition, display 414 operably connected to PVM 400 may be employed to show such output data.

In one embodiment, catheter 512 further comprises one or more distally-located pacing or electrical stimulation electrodes configured to electrically stimulate or pace the patient's heart with a pacing pulse while ablation energy is not being applied to the patient's endocardium or myocardium. On the basis of a point of origin of the evoked response caused by the pacing pulse that is calculated by EMS 10, EMS 10 generates a location corresponding to the distal end of catheter 512 within the patient's heart 16, which may be provided as a visual representation or image to the user of EMS 10.

Referring now to Fig. 9C, there is shown one non-invasive embodiment of a combined system utilizing EMS 10 and ablation system 500. By way of example, non-invasive external ablation system 50 may be a HIFU (High Intensity Focused Ultrasound) system, a proton beam system, X-rays (e.g., the CYBERKNIFE system) or any other system capable of delivering a focused beam of energy to target cardiac tissue and ablating such tissue controllably. As shown in Fig. 9C, ablation system 500 comprises display 520, computer/controller 502/550, ablation position controller 506, HIFU or other particle beam function generator 504, external non-invasive transducer 522 (which is configured to deliver a beam of tightly focused energy to a target location within or on the patient's heart 16 at focused beam or ablation location 538), and HIFU or other transducer positioner 524. Preferably, such a focused beam of energy is constrained to operate in a volume of cardiac tissue nor more than 3mm to 5mm in diameter (or even a smaller diameter, if technically achievable using transducer 522). Amplifier 532 and power meter 534 may be included in HIFU or other particle beam function generator 504. In a HIFU system, a plurality of imaging probes 528 may be employed to acquire ultrasonic backscatter and other data to image the patient's heart 16 and other organs or regions during the ablation procedure. A visualized representation of image of scar tissue 420 that has been formed in or on patient's heart 16, or a desired location of scar tissue in or on patient's heart 16, may be shown on display 520 or display 414. HIFU or other type of ablation beam 542 is guided using ablation position controller 506 and transducer positioner 524 such that the ablation energy delivered thereby is focused in the correct location within or on patient's heart 16. Further shown in Fig. 9C for illustrative purposes is location 22, which is the point of origin of atrial fibrillation in patient's heart 16. The scar location indicated by visualization or image 420 is intended to prevent the spread of arrhythmias originating at location 22 to other portions of the patient's heart.

In non-invasive embodiments of combined EP mapping and ablation system 10, the risk and disadvantages should be reduced relative to invasive methods. Consequently, morbidity would be expected to be decreased due to reduced secondary complications from invasive procedures such as infections. Non-invasive embodiments can also lead to shorter recovery times compared to hospitals stays from surgery or interventional procedures, with corresponding reduced costs.

Referring now to Fig. 10(a), there is shown a schematic representation of a patient's heart 16 having inserted therein ablation catheter 512 and EP sensing and/or stimulating catheters 544, 5467and 548. In Figs. 10(b) through 10(f), there are shown several illustrative visual representations or images of heart 16 that may be generated by system 10 during a combined EP mapping and cardiac ablation procedure.

In Fig. 10(b), an illustrative location 22 corresponding to a point of origin of atrial fibrillation or other arrhythmia in patient's heart 16 is shown, which may be included in visual representations or images of the electrical activity of the heart generated by system 10 and shown on a display to a user. Such visual representations or images can include depictions of electrical wavefronts emanating successively outward from arrhythmia point of origin 22 at times t₁ (422), t₂ (424), t₃ (426) and t₄ (428). See Fig. 10(b), where the arrhythmia signals spread outwardly from point 22 unimpeded. In Fig 10(c), ablation electrode 514 or ablation beam 538 is positioned at locations in heart 16 shown by icons or symbols 432 and 434, which may be included in visual representations or images of the electrical activity of the heart generated by system 10. As shown in Fig. 10(d), the scar location indicated by visualization or image 420 prevents the spread of arrhythmias originating at location 22 to some portions of the patient's heart. In Fig. 10(e), ablation electrode 514 or ablation beam 538 is positioned at locations in heart 16 shown by icons or symbols 436 and 438, which may be included in visual representations or images of the electrical activity of the heart generated by system 10. As shown in Fig. 10(e), the scar location indicated by visualization or image 420 now acts to prevent the spread of arrhythmias originating at location 22 to other portions of the patient's heart.

It will now be seen that combined EP mapping and ablation system 10 can provide real-time or near-real-time cardiac electrophysiological information, including sequences of cardiac excitation, zones with abnormal electrophysiological properties, locations of ectopic foci, drivers and triggers of arrhythmias, and possible targets for ablation.

### Computer Algorithm and Computer Algorithm Operation Examples

There are now described several different embodiments of computer algorithms and examples of corresponding computer pseudo-code that find application in the various methods, systems, devices and components described herein. Tables 1 through 8 below set forth various examples of such pseudo-code.

Referring first to Tables 1 and 2 below, registration methods (A) and (B) employ (X,Y,Z) or positional/spatial/Cartesian coordinate or location data corresponding to ablation catheter 512, which are provided by computer/ controller 502/500 (see Fig. 9B). Computer/controller 502 is configured to: (a) receive user inputs from the physician or other health care provider; (b) send to PVM 400/450 trigger signals and catheter position data; and (c) receive patient geometry data 302. See also, for example, U.S. Patent No. 7,715,604 to Sun et al. entitled "System and method for automatically registering three dimensional cardiac images with electro-anatomical cardiac mapping data".

To display the tip or other portion of ablation catheter 512 in the coordinate system being utilized by PVM 400/450 and/or MMU 200/250, or to provide to PVM 400/450 and/or MMU 200/250 with the spatial position or location where ablation energy has been applied or is being applied, in one embodiment the coordinate system used in ablation system 500 is registered with the coordinate system associated with patient geometry data 302. Two methods (A) and (B) are discussed below as embodiments of such a registration procedure, where the result provided is the position of the ablation catheter tip in heart 12 and its visualization in heart 12 in PVM 400/450 and/or MMU 200/250. Methods (A) and (B) may also be combined to improve registration accuracy.

In method (A) (see Table 1), landmarks (or fiducial marks) and labels are used for registration in conjunction with a positional receiving/transmitting type of catheter 512. In the embodiment of method (A), the computer program shown in Table 1 is executed on computer/controller 502/550 to perform the registration procedure. At the same time, the computer program shown in Table 2 is executed using the computer of PVM 400/450. The computer program of Table 1 stored in and executed by computer/controller 502/550 is linked to the computer program of Table 2 stored in and executed by PVM 400/450 such that the program of Table 2 receives catheter position data and trigger signals to initialize or terminate the display of the tip of catheter 512 on display 520 and/or display 414.

In method (B) (see Table 2), an evoked response of the heart is used for registration that is generated by a pacing electrode disposed near the tip of the catheter 512, and sensed by ablation system 500 and/or PVM 400/450. In the embodiment of method (B), the computer program shown in Table 3 is stored and executed on computer/controller 502 and/or PVM 400/450.

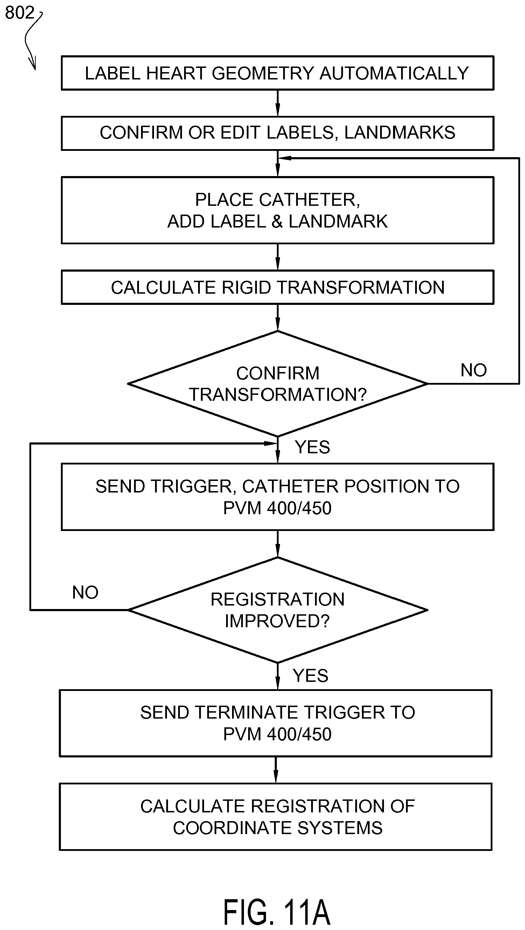

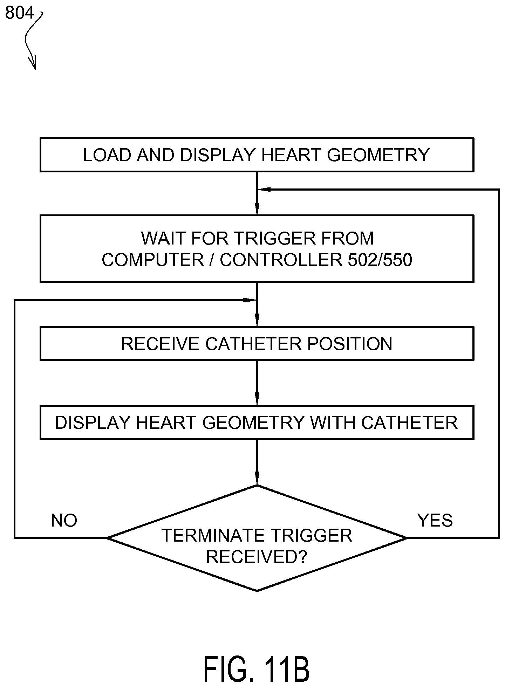

Referring to Figs. 9B, 11(a) and 11(b), the pseudo-code of Table 1 (which is stored in and executed by computer/controller 502/550), and the pseudo-code of Table 2 (which stored in an executed by the computer of PVM 400/450), where there is described one embodiment of a method and system for visualizing the location of the tip of an ablation catheter 512 in the heart 12, which is based on a system that utilizes a location receiving/transmitting type of ablation catheter.

In Table 1, computer/controller 502/550 initially loads heart geometry from patient geometry data 302. The heart geometry data is then processed automatically to label the endocardial surface and several anatomical landmarks. A repeat loop is then entered to confirm or edit the automatically generated labels and landmarks. Display 520 provides a visual display of the labels and landmarks. The physician is instructed to confirm the locations of the labels and landmarks that are displayed. Once the locations have been confirmed by the physician, the repeat loop terminates. Else, the loop continues and the physician is repetitively instructed to correct, add or remove landmarks, until he or she has confirmed them. In such a way, the computer visualizes on display 520 an interactive graphical user interface tool for repositioning and/or removal/ addition of labels and landmarks. Once the labels and landmarks have been finally confirmed, computer/controller 502/550 starts saving log object data corresponding to catheter tip positions at a frequency of 100 Hz. A repeat loop is then entered to repetitively improve the registration of coordinate systems for the display of the tip of catheter 512 using patient geometry data 302.

The repeat loop starts with an instruction on display 520 to place catheter 512 on any anatomical landmark or surface of the heart. The computer waits for the user/ physician to confirm the catheter tip is located at an anatomical landmark or surface of the heart. The current catheter tip position is then obtained from the log object and stored temporarily. Subsequently, computer/controller 502/550 instructs the physician via display 520 to select respective anatomical landmarks or labels of the heart from a displayed list of stored labels and landmarks. The temporarily stored catheter tip position in the coordinate system of computer/controller 502/550 is then stored with the selected label or landmark. Subsequently, and still in the repeat loop, computer/controller 502/550 calculates a rigid transformation matrix between its coordinate system and the coordinate system of patient geometry data 302.

In one embodiment, computer/controller 502 employs an optimization method comprising: ( a) an iterative-closest-point method for the selection of point correspondences between the stored labels and points that are automatically selected from the catheter tip positions in the log object; (b) a method that selects labels and landmarks where related catheter positions have been stored; and (c) a method that minimizes the mean squared transformation error of the point correspondences. The computer/controller 502/550 then shows on display 520 the combined visualization of the heart geometry data with the landmarks and labels, and the transformed positions of the ablation catheter tip which were previously assigned to the landmarks and labels. Subsequently, the physician is instructed on display 520 to confirm the computed transformations.

If the computed transformation is not confirmed, the repeat loop routine is repeated. If the computed transformation is confirmed, a trigger signal is sent to PVM 400/450 for initiation of display of the catheter tip, and a nested repeat loop is run until a command is received to "improve registration", which will cause the main loop to be repeated. While the nested repeat loop is running, computer/controller 502/550 calculates the position of the ablation catheter's tip in the coordinate system of patient geometry data 302 using the rigid transformation matrix and the latest catheter tip position. The position of the ablation catheter's tip is then sent to PVM 400/450, and the nested repeat loop continues until a button is pressed on display 520 to send a trigger signal for termination of catheter tip display to PVM 400/450 and to improve registration. See the accompanying flow chart for method 802 in Fig. 11(a).

**Table 1: Computer Pseudo-Code for Registration Method (A) Using Location Receiving/Transmitting Catheter 512**

| # Pseudo-code configured for execution by computer/controller 502/550 |
|---|
| |
| transfer_file('patient_geo.vtk', from = 'imaging_system300', |
| to = '.') |
| p_geo = load('patient_geo.vtk') |
| h_geo = extract_geo(p_geo, tissue = 'heart') |
| labels = label_endocardial_surface(h_geo) |
| landmarks = identify_landmarks(h_geo) |
| |
| % Confirm/ edit automatically generated labels, landmarks |
| repeat: |
| GUI.displayVTK(h_geo,labels,landmarks) |
| confirmed = (GUI.messagebox('Confirm |
| labels and landmarks?',{'Yes','Edit'})) |
| if confirmed == 'Yes': |
| break |
| else: |
| GUI.messagebox('Please correct, add or remove |
| labels or landmarks.',{'OK'}) |
| GUI.tool_manipulate({labels,landmarks}) |
| |
| log_c_xyz = cath_pos_logger.init(f_Hz=100) |
| % Calculate registration of coordinate systems for |
| % display of catheter |
| repeat: |
| % Obtain point correspondence from catheter placement |
| confirmed = (GUI.messagebox('Place catheter on landmark |
| or surface',{'Confirm position','Cancel'})) |
| if confirmed == 'Confirm position': |
| tmp_cath_pos = log_c_xyz.getpos() |
| selection = (GUI.selectfromlists('Select anatomical |
| landmark or label',labels,landmarks)) |
| add_point_correspondence(labels,landmarks,selection, |
| tmp_cath_pos) |
| else: |
| continue |
| % Calculate registration |
| res = inf |
| T = eye(4,4) |
| repeat until res < eps: |
| % point correspondences: select automatically |
| % from catheter position log |
| a_labels_pts, a_labels_w = ( |
| autoselect_labels_pts_from_log( |
| log_c_xyz, labels)) |
| pt_corresp_labels = ( |
| find_closest_points(labels.get_h_xyz(), |
| T, a_labels_pts)) |
| s_pts = a_labels_pts(pt_corresp_labels) |
| t_pts = labels.get_h_xyz(pt_corresp_labels) |
| w_pts = a_labels_w(pt_corresp_labels) |
| % point correspondences: labels |
| labels_cp, labels_w = labels_with_cath_pos(labels) |
| s_pts.append(labels.get_c_xyz(labels_cp)) |
| t_pts.append(labels.get_h_xyz(labels_cp)) |
| w_pts.append(labels_w) |
| % point correspondences: landmarks |
| landmarks_cp, landmarks_w = ( |
| landmarks_with_cath_pos(landmarks)) |
| s_pts.append(landmarks.get_c_xyz(landmarks_cp)) |
| t_pts.append(land marks.get_h_xyz(landmarks_cp)) |
| w_pts.append(landmarks_w) |
| % compute optimal transformation |
| T, res = mininize_mean_squared_error_T( |
| source_points = s_pts, |
| target_points = t_pts, |
| transformation = 'rigid', |
| weighting = w_pts) |
| % display result |
| GU I.displayVTK(h_geo,labels,landmarks,T) |
| confirmed = GUI.messagebox( |
| 'Confirm transformation?',{'Confirm','Improve'}) |
| if confirmed == 'Confirm': |
| send_trigger_signal(to = 'PVM 400/450', |
| type = 'init_cath_ display') |
| % allow PVM 400/450 to display catheter |
| repeat: |
| c_pos_p_geo = transform(T, log_c_xyz) |
| send_data(to = 'PVM 400/450', type = 'catheter_pos', |
| data = 'c_pos_p_geo') |
| event = GUI.eventbutton('improve registration') |
| if event == 'button_pressed': |
| send_trigger_signal(to = 'PVM 400/450', |
| type = 'terminate_cath_display') |
| break |
| else: |
| continue |

Referring to Table 2 below and to Fig. 11(b), at the same time computer program (A) of Table 1 is running on computer/controller 502/550, computer program (B) is running on PVM 400/450. Initially, the program in Table 2 loads patient geometry data 302 in PVM 400/450 and visualizes the surface of the heart on the computer screen of PVM 400/450. Then, it enters a repeat loop. While in the loop it first waits for the trigger signal for initiation of catheter tip display from computer/controller 502/550 and then receives the catheter tip position in the coordinate system of patient geometry data 302 from computer/controller 502/550. Second, it runs a nested loop to display the catheter tip along with the surface of the heart on the computer screen of PVM 400/450. In that nested loop, it then checks whether a trigger signal for termination of catheter tip display has been received from computer/controller 502/550 and terminates the nested repeat loop in that case . Otherwise, the nested loop is continued and a new catheter tip position is received from computer/controller 502/550. See the accompanying flow chart for method 804 in Fig. 11(b).

**Table 2: Computer Pseudo-Code for Registration Method (B) Using Location Receiving/Transmitting Catheter 512**

| # Pseudo-code configured for execution by the computer of PVM 400/450 |
|---|
| |
| transfer_file('patient_geo.vtk', |
| from = 'patient_geometry_data_302', to = '.') |
| p_geo = load('patient_geo.vtk') |
| h_geo = extract_geo(p_geo, tissue = 'heart') |
| |
| GUI.displayVTK(h_geo) |
| |
| repeat: |
| wait_for_trigger_signal(from = 'computer/controller 502/550', |
| type = 'init_cath_ display') |
| catheter_pos = wait_for_data( |
| from = 'computer/controller 502/550', |
| type = 'catheter_pos', |
| data = 'c_pos_p_geo') |
| repeat: |
| GUI.displayVTKcath(h_geo, catheter_pos) |
| check = check_for_receipt_of_trigger_signal( |
| from = 'computer/controller 502/550', |
| type = 'termiante_cath_display') |
| if check == 'received': |
| break |
| else: |
| catheter_pos = wait_for_data( |
| from = 'computer/controller 502/550', |
| type = 'catheter_pos', data = 'c_pos_p_geo') |

Referring to Figs. 7, 9B, and 11(c), and also to the pseudo-code of Table 3 below (which is configured for execution on the computer of PVM 400/450), another embodiment of the visualization of an ablation catheter tip position in the heart is described, which is based on an evoked response generated by a pacing electrode disposed near or at the tip of the catheter 512, which is sensed by ablation system 500 or other potion of EMS 10. Initially, EMS 10 performs steps 614, 616, 618 of Fig. 7. Then, the physician stimulates myocardial tissue with a pacing electrode included in catheter 512.

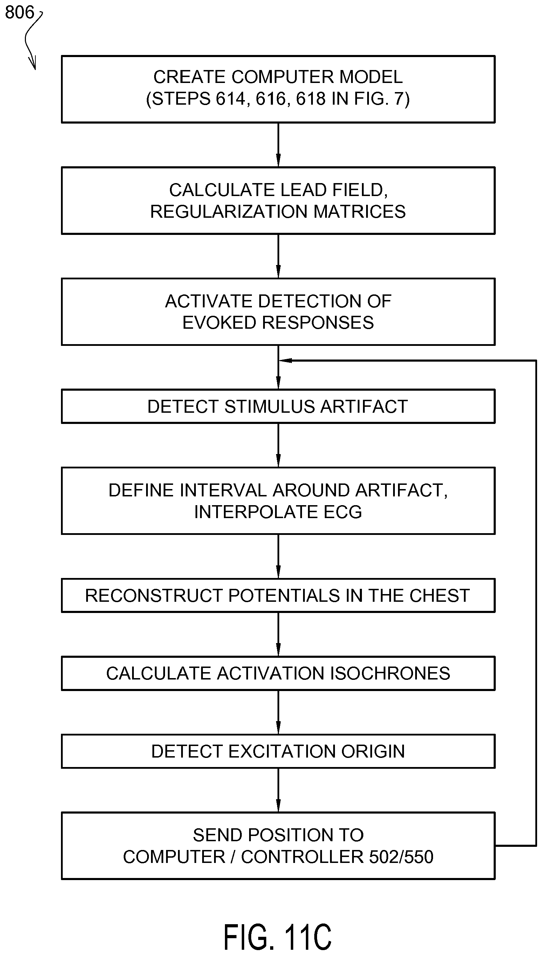

Once the detection algorithm has been activated, EMS 10/ablation system 500 repetitively detects stimulus artifact ECG signals during real-time ECG processing step 608 in Fig. 7. The algorithm of Table 3 then defines a time interval with respect to the stimulus artifact in step 612 of Fig. 7, and performs ECG interpolation to produce an isopotential map on the torso in step 620 of Fig. 7. Subsequently, the algorithm of Table 3 reconstructs potentials on the epicardial and endocardial surfaces shown in step 622 of Fig. 7. Further, the system produces an isochronous map of a the heart model based on patient geometry data 302, and detects the excitation origin in the isochronous map with respect to the coordinate system of patient geometry data 302. Finally, the excitation origin coordinates are sent to computer/controller 502/550 of the ablation system 500. Optionally, computer/ controller 502/550 of ablation system 500 uses received coordinates and point correspondences between the received excitation origin and the catheter tip position to calculate or improve the calculation of a transformation matrix between the coordinate system of patient geometry data 302 and the coordinate system of ablation system 500 (see also Table 2). See the accompanying flow chart for method 806 in Fig. 11(c).

**Table 3: Computer Pseudo-Code for Evoked Response Origin Detection**

| # Pseudo-code configured for execution by the computer 400/450 |
|---|
| |
| % Initialize model |
| imaging_data = load_CT_imaging_data() |
| model_vox = GUI.tool_create_voxel_model(imaging_data) |
| model_poly = Mesher(model_vox) |
| el_coords = DetectElectrodeCoordinates(model_poly,model_vox) |
| GUI.displayVTK(model_poly,el_coords) |
| LF, R = calculate_leadfield_matrices(model_poly,el_coords) |
| |
| GUI.messagebox('Activate detection of evoked responses of pacing electrodes?',{'OK'}) |
| |
| repeat: |
| % Real-time ECG processing |
| repeat until detected == True: |
| ecg_data = process_ECG_RT() |
| stim_t, detected = detect_stimulus_artifact(ecg_data) |
| ecg_stim = process_ECG(begin = stim_t-30, end = stim_t+70, ecg_data) |
| ecg_stint = interpolate(ecg_stim, model_poly) |
| pot_endo_epi_stim = solve_inverse(ecg_stint, LF, R) |
| ISOCHRs = calculate_ISOCHR(pot_endo_epi_stim) |
| xyz_stim = detect_excitation_origin(ATs,model_poly) |
| send_trigger_data(to = 'computer/controller 502/550', value = xyz_stim) |

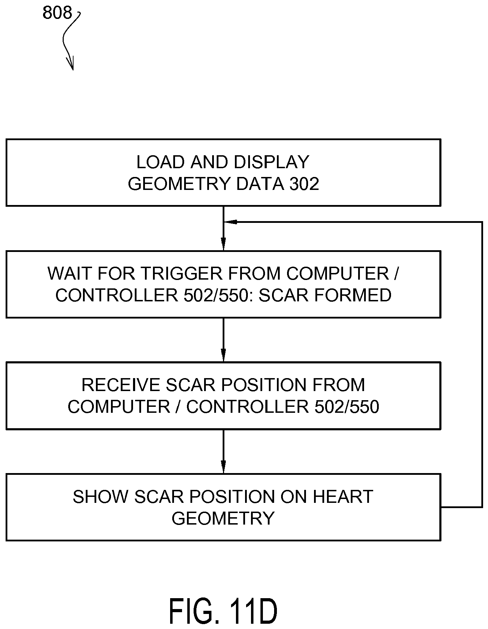

In one embodiment, a visual representation of one or more ablation scars is generated for display 4141 using the computer of PVM 400/450 in accordance with the generalized scar visualization algorithm set forth in Table 4 below (and as further illustrated in the flow chart of Fig. 11(d)). Once a scar has been formed, PVM 400/450 receives a trigger signal from computer/controller 502/550 along with the position of the ablation scar, which can correspond to a catheter tip position in the coordinate system of the patient geometry data 302 (as derived using the method of Table 1 and/or Table 2 above, or as derived from the position of a scar using the method of Table 4).

Referring to Table 4 below, and to Figs. 2, 9B, 9C, and to method 808 of Fig. 11(d), initially, the computer of PVM 400/450 loads heart geometry from patient geometry data 302 and displays same on display 414 of PVM 400/450. Then, a repeat loop is initiated that waits for computer/controller 502/550 to send a trigger signal that indicates a scar has been formed. Subsequently, the position of the scar in the coordinate system of the patient geometry data 302 is received from computer/controller 502/550, and a marker and the heart are on display 414 of PVM 400/450.

**Table 4: Generalized Computer Pseudo-Code for Visualizing Ablation Scars on the Heart**

| # Pseudo-code configured for execution by the computer of PVM 400/450 |
|---|
| |
| transfer_file('patient_geo.vtk', from = 'patient_geometry_data_302', to = '.') |
| p_geo = load('patient_geo.vtk') |
| h_geo = extract_geo(p_geo, tissue = 'heart') |
| |
| GUI.displayVTK(h_geo) |
| |
| % Display scars |
| repeat: |
| wait_for_trigger_signal(from = 'computer/controller 502/550', |
| type = 'scar_formed') |
| scar_pos = wait_for_data(from = 'computer/controller 502/550', |
| type = 'scar_pos', |
| data = 'scar_pos_p_geo') |
| GUI.displayMarker(h_geo,scar_pos) |

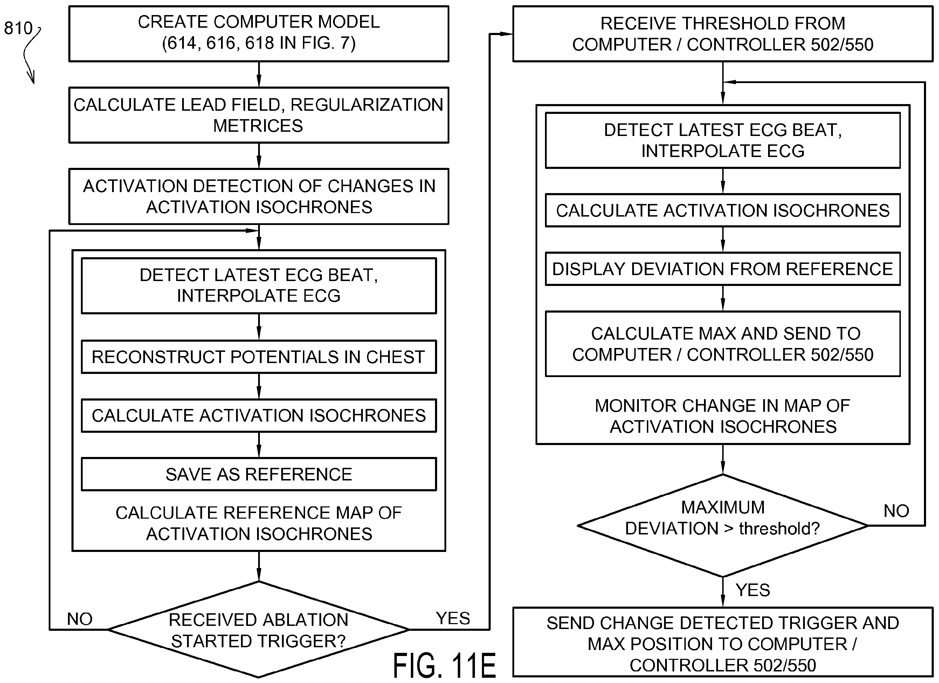

Referring to Table 5 below and to method 810 of Fig. 11(e), an alternative algorithm for generating visual representations of ablation scars on display 414 of PVM 400/450 is shown, where the computer program of Table 5 is stored in and executed by the computer of PVM 400/450. Once a scar has been formed, the algorithm of Table 5 receives a trigger signal from computer/controller 502/550 along with the position of the ablation scar (which may be the position of the catheter tip in the coordinate system of patient geometry data 302 as determined using method (A) or method (B) above (Tables 1 and 2, respectively), or using the method of Table 5 below). Referring to Table 5, Fig. 9B, and Fig. 11(e), initially, the computer of PVM 400/450 loads a heart geometry file or data from patient geometry data 302, which is shown on display 414 of PVM 400/450. Then, a repeat loop is started that waits for computer/controller 502/550 to send a trigger signal that indicates that a scar has been formed. Subsequently, the position of the scar in the coordinate system of patient geometry data 302 is received from computer/controller 502/550 by PVM 400/450, and a marker is shown to the user or physician at the determined scar location or position along with heart 12 on display 414 of PVM 400/450.

The pseudo-code of Table 5 also includes the detection of changes that are greater than a predetermined threshold, sending trigger signals to and receipt of trigger signals from ablation system 500 at the start of ablation, detection of changes in activation isochrones, and/or resets of such isochrones change detections. Initially, PVM 400/450 in combination with ablation system 500 performs steps 614, 616, 618 of Fig. 7. Then, the physician is queried through a user interface to activate the detection of changes in activation isochrones. This starts a repeat loop, in which a reference map of activation isochrones is repeatedly calculated in a nested repeat loop, until a signal is received from computer/controller 502/550 that indicates that ablation has started. The ECG interval corresponding to the latest 10 seconds of data is repeatedly loaded in step 612 in Fig. 7 from the real-time ECG processing data that is provided in step 608 in Fig. 7. From the ECG interval, the latest heart beat is then automatically detected, and the ECG of the beat is interpolated to produce an isopotential map on the torso in step 620 of Fig. 7. Subsequently, the computer program of Table 4 reconstructs potentials on the epicardial and endocardial surfaces in step 622 of Fig. 7. System 10 produces a map of activation isochrones of a heart model that is based on patient geometry data 302. This map is saved as a reference map of activation isochrones. Once a signal is received from computer/controller 502/550 indicating that ablation has started, the nested loop terminates and a threshold value is received from computer/controller 502/550. Using this threshold value, the change in the map of activation isochrones is then monitored in a subsequent nested loop. As described above, to monitor a change in an isochronal activation map, current and updated isochronal maps are continuously and repeatedly calculated using the latest heartbeat. In the nested repeat loop, the difference of the current isochronal map with respect to its reference map is then computed and displayed on display 414 of PVM 400/450. The computer of PVM 400/450 then calculates the maximum difference between the current map of activation isochrones and the reference map, and sends this information to computer/controller 502/550.

Once the maximum difference has crossed the previously received threshold value, a trigger signal is sent to computer/controller 502/550 which indicates that a change has been detected in the map of activation isochrones. Further, the position of the index of the maximum change in the heart model is identified and its location is sent to the computer/controller 502/550.

To re-initiate the detection of changes that are related to scar formation, the system then waits for the user to confirm a message that is shown on the display of PVM 400/450 to break the nested loop and continue with the loop of continuously calculating a reference map. The computer/controller 502/550 is notified of the reset by a trigger message. See the accompanying flow charts for method 810 in Fig. 11(e).

**Table 5: Computer Pseudo-Code for Visualizing Ablation Scars on the Heart**

| # Pseudo-code configured to be executed by the computer of PVM 400/450 |
|---|
| |
| % Initialize model |
| imaging_data = load_CT_imaging_data() |
| model_vox = GUI.tool_create_voxel_model(imaging_data) |
| model_poly = Mesher(model_vox) |
| el_coords = DetectElectrodeCoordinates(model_poly,model_vox) |
| GUI.displayVTK(model_poly,el_coords) |
| LF, R = calculate_leadfield_matrices(model_poly,el_coords) |
| |
| GUI.messagebox('Activate detection of change in activation isochrones?',{'OK'}) |
| |
| repeat: |
| % Save reference map of activation isochrones |
| repeat: |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| ref_ISOCHRs = calculate_ISOCHR(pot_endo_epi) |
| received = (check_if_signal_received( |
| from = 'computer/controller 502/550', |
| type = 'ablation_started')) |
| if received == 'ablation_started': |
| % Receive threshold set for control of ablation system |
| threshold = receive_data(from = 'computer/controller 502/550', type = 'act_iso_threshold') |
| |
| break |
| else: |
| continue |
| % Once ablation has started, monitor change in map |
| repeat: |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| cur_ISOCHRs = calculate_ISOCHR(pot_endo_epi) |
| |
| act_iso_diff = cur_ISOCHRs-ref_ISOCHRs |
| i, max = max(act_iso_diff) |
| if max > threshold: |
| send_trigger_signal(to = 'computer/controller 502/550', |
| value = 'change_in_act_iso_detected') |
| max_pos = model_poly.getxyz(heart_id = i) |
| send_data(to = 'computer/controller 502/550', |
| type = 'scar_pos', data = max_pos) |
| send_data(to = 'computer/controller 502/550', |
| type = 'act_iso_maxdiff', data = max) |
| GUI.displayVTK(model_poly,act_iso_diff) |
| GUI.messagebox('Reset and restart detection of change in activation isochrones?',{'OK'}) |
| send_trigger_signal(to = 'computer/controller 502/550', |
| value = 'change_in_act_iso_reset') |
| break |
| else: |
| send_data(to = 'computer/controller 502/550', |
| type = 'act_iso_maxdiff', data = max) |
| GUI.displayVTK(model_poly,act_iso_diff) |

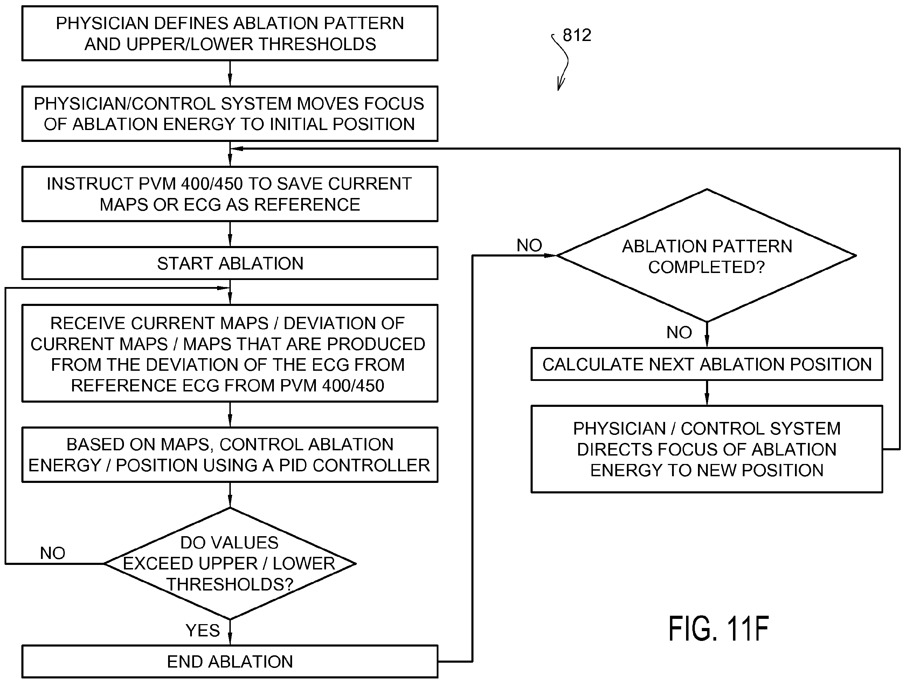

Referring to Table 6 below and to method 812 of Fig. 11(f), an algorithm for closed-loop control of an ablation device is set forth, where the computer program of Table 6 is stored in and executed by controller/computer 502/550 of ablation system 500 operating in conjunction with PVM 400/450.

To facilitate closed-loop control of an ablation device through imaging data provided by PVM 400/450 and/or imaging system 300, the computer program of Table 6 is run on computer/controller 502/550 of ablation system 500. In the following discussion regarding the pseudo-code of Table 5, please refer to Figs. 7, 9B, 9C and the flow chart of Fig. 11(f).

Initially, the computer program of Table 6 loads patient geometry data 302 and starts a parent repeat loop, which performs the following steps. In a graphical user interface of ablation system 500, the physician or other user defines an ablation pattern on the heart model, which may be a point, line, area, volume, or any combination thereof, and the user also defines upper and/or lower thresholds for termination of ablation. Next, either the user or physician is instructed via display 414 to move the catheter tip to the initial position of the defined ablation pattern, or position controller 506 of the ablation system moves the focus of ablation energy (provided, for example, by transducer 522 of Fig. 9C) to an initial position of the defined ablation pattern. A nested repeat loop then begins to execute ablation of the defined ablation pattern. In the nested repeat loop, the program of Table 6 instructs PVM 400/450 to save the current heart maps and/ or ECG as references, and requests physician or user confirmation to start ablation. Upon confirmation, RF generator, HIFU, or particle beam function generator 504 is given a command to perform ablation. A nested repeat loop then starts to control scar formation in a closed loop in conjunction with PVM 400/450. In the nested repeat loop for scar formation, the program of Table 6 receives from PVM 400/450 the current heart maps or derivations of current heart maps from the reference heart maps, or heart maps that are derived from one or more ECGs or a reference ECG.

The received data, along with the current position of the tip of catheter 512 or the energy beam focal point 538 for transducer 522, is then input into a proportional-integral-derivative controller (PID controller), or any other suitable controller or processor forming a portion of computer/controller 502/550, to produce a corrected ablation catheter tip or energy beam position in or on the patient's heart. Other parameters affecting the location, duration and energy provided by ablation system to patient's heart 14 may also serve as inputs to the PID controller. Data relating to the catheter tip or energy beam position 538 and other parameters are then provided to RF generator, HIFU, or particle beam function generator 504 and/or to the ablation position controller 506. The program of Table 6 then determines whether any of the current heart maps or derivation of current heart maps from reference heart or other maps derived from ECGs or reference ECGs exceed any of predetermined thresholds, including thresholds that have been set for values that are derived from heart maps (e.g., maxima, minima, averages, values at current or previous ablation positions, etc.). If thresholds are not exceeded, the nested scar formation repeat loop is continued. Else, if thresholds are exceeded, RF generator, HIFU, or particle beam function generator 504 is given a command to terminate ablation. Then, the nested repeat loop for scar formation is terminated, and a determination is made whether the ablation pattern has been completed. If so, the nested ablation pattern repeat loop is terminated and a new ablation pattern may be defined in the parent repeat loop. Else, the next ablation position is calculated based on the defined ablation pattern, and either the physician or other user is instructed via a display to move the catheter tip to that position, or position controller 506 of ablation system 500 moves the focus of ablation energy to the next ablation position. See the flow chart for method 812 of Fig. 11(f).

**Table 6: Computer Pseudo-Code for Closed-Loop Ablation Control**

| # Pseudo code configured for execution on computer/controller 502/550 |
|---|
| |
| %Initialize |
| |
| p_geo = load('patient_geo.vtk') |
| h_geo = extract_geo(p_geo, tissue = 'heart') |
| |
| repeat: |
| GUI.messagebox(Define ablation pattern',{'OK'}) |
| pattern = GUI.tool_specify_ablation_pattern(h_geo) |
| GUI.messagebox('Set upper and lower thresholds for termination of ablation',{'OK'}) |
| tresholds = GUI.tool_set_thresholds() |
| pos = pattern.get_init_pos() |
| position_controller.set_ablation_position(pos) |
| % perform ablation of defined pattern |
| repeat: |
| send_trigger(to = 'PVM 400/450', |
| type = 'save maps and ECG as reference') |
| confirm = GUI.messagebox('Confirm to start ablation',{'OK'}) |
| 504.start_ablation() |
| % control scar formation |
| repeat: |
| cur_maps = receive_data(from = 'PVM 400/450', type = 'cur_maps') |
| dev_cur_maps_from_ref_maps = receive_data(from ='PVM 400/450', type = 'dev_cur_maps_from_ref_maps') |
| maps_dev_ecg_from_ref = receive_data(from ='PVM 400/450', |
| type = 'maps_dev_ecg_from_ref) |
| cur_pos = position_controller.get_position() |
| energy_params, position = PID_controller(pos, cur_pos, tresholds) |
| 504.set_ablation_energy(energy_params) |
| position_controller.set_ablation_position(position) |
| if tresholds_exceeded(cur_maps, dev_cur_maps_from_ref_maps, maps_dev_ecg_from_ref, tresholds): |
| 504.terminate_ablation() |
| break |
| else: |
| continue |
| % proceed with next position in ablation pattern |
| n_pos = next_position(pos, pattern) |
| if n_pos == None: |
| break |
| else: |
| position_controller.set_ablation_position(n_pos) |

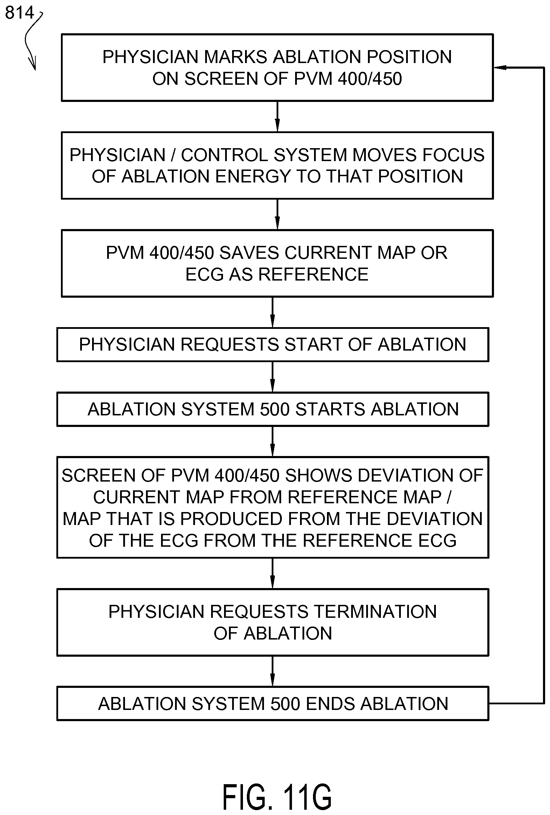

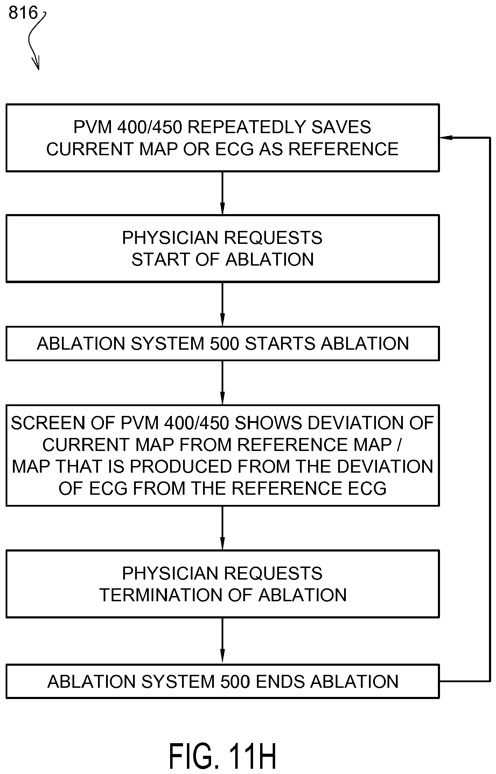

Referring to Tables 7 and 8 below, and to method 814 of Fig. 11(g) and method 816 of Fig. 11(h), two different embodiments of algorithms for defining ablation positions and monitoring scar formation are shown. Table 7 shows computer pseudo-code for an algorithm that permits a user to define ablation positions and monitor scar formation. Table 8 shows computer pseudo-code for an algorithm where only ablation-related scar formation is monitored, but a display or screen is not used to define ablation positions. In the pseudo-code of Table 8, a user interface is employed to provide instructions to ablation system 500 to start or end ablation in a loop that facilitates the monitoring of scar formation. In the pseudo-code of Table 7, a user interface is employed that in addition to providing instructions to start or end ablation, also provides instructions to define the ablation positions via the user interface, which then directs the focus of ablation energy to the defined ablation position(s).

Referring to Table 7 below, and to Figs. 2, 7, 9B, 9C, and 11(g), a user interface is employed to define ablation positions and monitor scar formation in an embodiment where the computer program is run on the computer of PVM 400/450. The user interface permits the focus, position/location and initiation/termination of ablation energy delivered to the patient's heart 14 to be controlled by the user via an appropriate user interface (e.g., graphical user interface, or GUI). Initially, PVM 400/450 performs steps 614, 616, 618 in Fig. 7. Then, an event button object is created and the physician or other user is requested via the user interface to activate the monitoring of ablation-related changes in activation isochrones. This starts a parent repeat loop, in which the GUI first provides an interface which allows the physician to mark an ablation position on a visual representation of the heart 14 or a portion of the heart. Next, either the physician or other user is instructed via display 414 and/or 520 to move the catheter tip or energy beam focal point to a subsequent ablation position. Alternatively, position controller 506 of the ablation system is employed to move the focus of ablation energy to the next ablation position.

A reference map of activation isochrones of the patient's heart is then repeatedly calculated in a nested repeat loop until an event button is clicked to start ablation. The ECG interval of the latest 10 seconds of time is repeatedly loaded in step 612 of Fig. 7 from the real-time ECG processing data that has been provided in step 608. From the ECG interval, the latest heart beat is then automatically detected, and the ECG of the beat is interpolated to produce an isopotential map of the torso in step 620 of Fig. 7. Subsequently, the computer program of Table 6 reconstructs potentials for the epicardial and endocardial surfaces in step 622 of Fig. 7. System 10 then produces a map of activation isochrones according to a heart model that is based on patient geometry data 302. This map is saved as a reference map of heart activation isochrones. Once the event button has been clicked to start ablation, RF generator, HIFU, or particle beam function generator 504 is given a command to perform ablation and the nested loop terminates. An interrupt is then defined for the event button in case it is clicked to terminate ablation.

As described above, and to monitor changes in the map of activation isochrones, a current isochronal map is repeatedly calculated from the latest heartbeat. In this nested repeat loop, differences between the current isochronal map and the reference map are computed and displayed on display 414 of PVM 400/450. The repeat loop is immediately terminated once an interrupt is received from the event button to terminate ablation, and RF generator, HIFU, or particle beam function generator 504 is given a command to terminate ablation.

To re-initiate the monitoring of ablation-related changes in activation isochrones, the GUI continues in the parent repeat loop for definition of a new ablation position. See the flow chart for method 814 in Fig. 11(g).

**Table 7: Computer Pseudo-Code for Defining Ablation Positions and Monitoring Scar Formation**

| # Pseudo-code configured to be executed by the computer of PVM 400/450 |
|---|
| |
| % Initialize model |
| imaging_data = load_CT_imaging_data() |
| model_vox = GUI.tool_create_voxel_model(imaging_data) |
| model_poly = Mesher(model_vox) |
| el_coords = DetectElectrodeCoordinates(model_poly,model_vox) |
| GUI.displayVTK(model_poly,el_coords) |
| LF, R = calculate_leadfield_matrices(model_poly,el_coords) |
| |
| event_bt = GUI.eventbutton.create() |
| GUI.messagebox('Activate monitoring of ablation-related change in activation |
| isochrones?',{'OK'}) |
| repeat: |
| pos = GUI.tool_mark_ablation_position(model_poly) |
| position_controller.set_ablation_position(pos) |
| % Save reference map of activation isochrones |
| event_bt = 'Start ablation' |
| event_bt.clicked = False |
| repeat: |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| ref_ISOCHRs = calculate_ISOCHR(pot_endo_epi) |
| event_bt.show() |
| if event_bt.clicked == True: |
| 504.start_ablation() |
| break |
| else: |
| continue |
| event_bt = 'Stop ablation' |
| event_bt.clicked = False |
| Buttonlnterrupt = event_bt.createInterrupt() |
| try: |
| % Once ablation has started, monitor change in map |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| cur_ISOCHRs = calculate_ISOCHR(pot_endo_epi) |
| |
| act_iso_diff = cur_ISOCHRs-ref_ISOCHRs |
| GUI.displayVTK(model_poly,act_iso_diff) |
| except ButtonInterrupt: |
| 504.terminate_ablation() |
| break |

Referring to Table 8 below, and to Figs. 2, 7, 9B, 9C and 11(h), a user interface is employed to monitor scar formation and is used to instruct ablation system 500 to start and end ablation. Initially, PVM 400/450 performs steps 614, 616, 618 in Fig. 7. Then, an event button object is created and the physician is requested via the user interface to activate the monitoring of ablation-related changes in activation isochrones. This starts a parent repeat loop, in which a reference map of heart activation isochrones is repeatedly calculated in a nested repeat loop until an event button is clicked by the physician or other user to start ablation. The ECG interval corresponding to the of the latest 10 seconds of data is repeatedly loaded in step 612 of Fig. 7 from the real-time ECG processing data that has been provided in step 608 of Fig. 7.

From the ECG interval, the latest heart beat is then automatically detected, and the ECG of the beat is interpolated to produce an isopotential map on torso 12 in step 620 of Fig. 7. Subsequently potentials corresponding to the epicardial and endocardial surfaces in step 622 of Fig. 7 are reconstructed. System 10 produces a map of activation isochrones for a heart model that is based on patient geometry data 302. This map is saved as a reference map of heart activation isochrones. Once the event button has been clicked to start ablation, RF generator, HIFU, or particle beam function generator 504 is given a command to perform ablation and the nested loop terminates. An interrupt is then defined for an event button in case it is clicked to terminate ablation.

As described above, to monitor changes in the activation isochrones maps, a current isochronal map is repeatedly calculated using the latest heartbeat. In this nested repeat loop, differences between the current isochronal map and the reference map are computed and displayed on display 414 of PVM 400/450. The repeat loop is immediately terminated once an interrupt is received from the event button to terminate ablation and RF generator, HIFU, or particle beam function generator 504 is given the command to terminate ablation.

To re-initiate the monitoring of ablation-related changes in activation isochrones, system 10 continues in the parent repeat loop and repeatedly calculates a reference map until a new command is received to start ablation. See method 816 in the flow chart of Fig. 11(h).

**Table 8: Computer Pseudo-Code for Monitoring Scar Formation**

| # Pseudo-code configured to be executed by the computer of PVM 400/450 |
|---|
| |
| % Initialize model |
| imaging_data = load_CT_imaging_data() |
| model_vox = GUI.tool_create_voxel_model(imaging_data) |
| model_poly = Mesher(model_vox) |
| el_coords = DetectElectrodeCoordinates(model_poly,model_vox) |
| GUI.displayVTK(model_poly,el_coords) |
| LF, R = calculate_leadfield_matrices(model_poly,el_coords) |
| event_bt = GUI.eventbutton.create() |
| GUI.messagebox('Activate monitoring of ablation-related change in activation isochrones?',{'OK'}) |
| repeat: |
| % Save reference map of activation isochrones |
| event_bt = 'Start ablation' |
| event_bt.clicked = False |
| repeat: |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| ref_ISOCHRs = calculate_ISOCHR(pot_endo_epi) |
| event_bt.show() |
| if event_bt.clicked == True: |
| 504.start_ablation() |
| break |
| else: |
| continue |
| event_bt = 'Stop ablation' |
| event_bt.clicked = False |
| ButtonInterrupt = event_bt.createInterrupt() |
| try: |
| % Once ablation has started, monitor change in map |
| ecg_last_10s = process_ECG_RT(return_last_ms = 10000) |
| ecg_beat = detect_last_beat(ecg_last_10s) |
| ecg_stint = interpolate(ecg_beat, model_poly) |
| pot_endo_epi = solve_inverse(ecg_stint, LF, R) |
| cur_ISOCHRs = calculate_ISOCHR(pot_endo_epi) |
| |
| act_iso_diff = cur_ISOCHRs-ref_ISOCHRs |
| GUI.displayVTK(model_poly,act_iso_diff) |
| except ButtonInterrupt: |
| 504.terminate_ablation() |
| break |

Referring to Fig. 7, it is to be understood that not only is it possible to monitor electrophysiological changes in the heart by comparing maps in step 610 over time, but it is also possible to compute changes in the ECG over time, and to monitor maps of electrophysiological changes in the heart or ECG and use such maps as inputs to steps 612 or 608. While in some embodiments, such as the algorithms presented in Tables 6, 7 and 8, methods are described where a current heart map of step 610 is produced and compared to a reference map in step 610, it is also possible to save and compare real-time ECGs from step 608, retrospective ECGs from step 612, or interpolated ECGs from step 620 as reference maps. Then, to produce maps of electrophysiological changes in the heart, the deviation of the current ECG from the reference ECG can be computed, and a map according to step 622 can be reconstructed showing changes in potentials in the heart, which in turn may be employed to produce a variant of a map produced in 610 that represents changes in electrophysiological properties of heart 14.

In one embodiment, scar-related ST segment elevation in the ECG may be obtained from differences between the current ECG and a reference ECG. Then, as in step 622 of Fig 7, system 10 reconstructs scar-related changes of potentials in the chest or torso, and a scar map of electrophysiological changes in the heart is produced as another variant of step 610 in Fig 7.

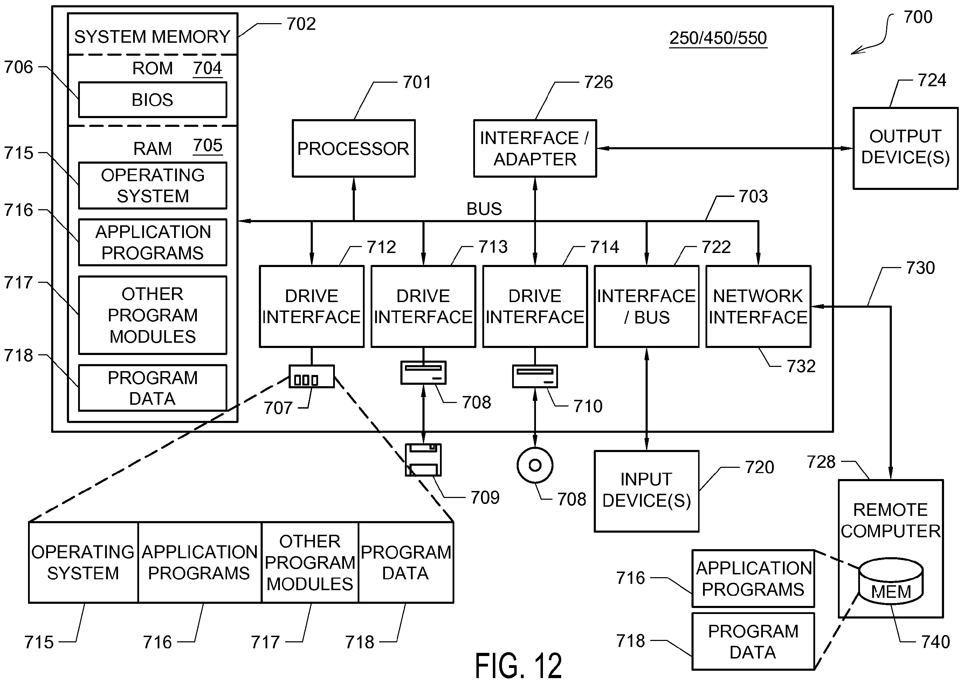

In view of the structural and functional descriptions provided herein, those skilled in the art will appreciate that portions of the described devices and methods may be configured as methods, data processing systems, or computer algorithms. Accordingly, these portions of the devices and methods described herein may take the form of a hardware embodiment, a software embodiment, or an embodiment combining software and hardware, such as shown and described with respect to the computer system of Fig. 12. Furthermore, portions of the devices and methods described herein may be a computer algorithm stored in a computer-usable storage medium having computer readable program code on the medium. Any suitable computer-readable medium may be utilized including, but not limited to, static and dynamic storage devices, hard disks, optical storage devices, and magnetic storage devices.

Certain embodiments of portions of the devices and methods described herein are also described with reference to block diagrams of methods, systems, and computer algorithm products. It will be understood that such block diagrams, and combinations of blocks diagrams in the Figures, can be implemented using computer-executable instructions. These computer-executable instructions may be provided to one or more processors of a general purpose computer, a special purpose computer, or any other suitable programmable data processing apparatus (or a combination of devices and circuits) to produce a machine, such that the instructions, which executed via the processor(s), implement the functions specified in the block or blocks of the block diagrams.

These computer-executable instructions may also be stored in a computer-readable memory that can direct a computer or other programmable data processing apparatus to function in a particular manner, such that the instructions stored in the computer-readable memory result in an article of manufacture including instructions which implement the function specified in an individual block, plurality of blocks, or block diagram. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the an individual block, plurality of blocks, or block diagram.

In this regard, Fig. 12 illustrates only one example of a computer system 700 (which, by way of example, can be first computer or computer workstation 250, second computer or computer workstation 450, ablation system control 502, or any combination of the foregoing computers or computer workstations) that can be employed to execute one or more embodiments of the devices and methods described and disclosed herein, such as devices and methods configured to acquire and process sensor data, to process image data, and/or transform sensor data and image data associated with the analysis of cardiac electrical activity and the carrying out of the combined electrophysiological mapping and analysis of the patient's heart 16 and ablation therapy delivered thereto. Computer system 700 can be implemented on one or more general purpose computer systems or networked computer systems, embedded computer systems, routers, switches, server devices, client devices, various intermediate devices/nodes or standalone computer systems. Additionally, computer system 700 or portions thereof may be implemented on various mobile devices such as, for example, a personal digital assistant (PDA), a laptop computer and the like, provided the mobile device includes sufficient processing capabilities to perform the required functionality.

In one embodiment, computer system 700 includes processing unit 701 (which may comprise a CPU, controller, microcontroller, processor, microprocessor or any other suitable processing device), system memory 702, and system bus 703 that operably connects various system components, including the system memory, to processing unit 701. Multiple processors and other multi-processor architectures also can be used to form processing unit 701. System bus 703 can comprise any of several types of suitable bus architectures, including a memory bus or memory controller, a peripheral bus, or a local bus. System memory 702 can include read only memory (ROM) 704 and random access memory (RAM) 705. A basic input/output system (BIOS) 706 can be stored in ROM 704 and contain basic routines configured to transfer information and/or data among the various elements within computer system 700.

Computer system 700 can include a hard disk drive 707, a magnetic disk drive 708 (e.g., to read from or write to removable disk 709), or an optical disk drive 710 (e.g., for reading CD-ROM disk 711 or to read from or write to other optical media). Hard disk drive 707, magnetic disk drive 708, and optical disk drive 710 are connected to system bus 703 by a hard disk drive interface 712, a magnetic disk drive interface 713, and an optical drive interface 714, respectively. The drives and their associated computer-readable media are configured to provide nonvolatile storage of data, data structures, and computer-executable instructions for computer system 700. Although the description of computer-readable media above refers to a hard disk, a removable magnetic disk and a CD, other types of media that are readable by a computer, such as magnetic cassettes, flash memory cards, digital video disks and the like, in a variety of forms, may also be used in the operating environment; further, any such media may contain computer-executable instructions for implementing one or more parts of the devices and methods described and disclosed herein.

A number of program modules may be stored in drives and RAM 707, including operating system 715, one or more application programs 716, other program modules 717, and program data 718. The application programs and program data can include functions and methods programmed to acquire, process and display electrical data from one or more sensors, such as shown and described herein. The application programs and program data can include functions and methods programmed and configured to process data acquired from a patient for assessing heart function and/or for determining parameters for delivering a therapy, such as shown and described herein with respect to Figs. 1-10(f).

A health care provider or other user may enter commands and information into computer system 700 through one or more input devices 720, such as a pointing device (e.g., a mouse, a touch screen, etc.), a keyboard, a microphone, a joystick, a game pad, a scanner, and the like. For example, the user can employ input device 720 to edit or modify the data being input into a data processing algorithm (e.g., only data corresponding to certain time intervals). These and other input devices 720 may be connected to processing unit 701 through a corresponding input device interface or port 722 that is operably coupled to the system bus, but may be connected by other interfaces or ports, such as a parallel port, a serial port, or a universal serial bus (USB). One or more output devices 724 (e.g., display, a monitor, a printer, a projector, or other type of display device) may also be operably connected to system bus 703 via interface 726, such as through a video adapter.

Computer system 700 may operate in a networked environment employing logical connections to one or more remote computers, such as remote computer 728. Remote computer 728 may be a workstation, a computer system, a router, , or a network node, and may include connections to many or all the elements described relative to computer system 700. The logical connections, schematically indicated at 330, can include a local area network (LAN) and/or a wide area network (WAN).

When used in a LAN networking environment, computer system 700 can be connected to a local network through a network interface or adapter 732. When used in a WAN networking environment, computer system 700 may include a modem, or may be connected to a communications server on the LAN. The modem, which may be internal or external, can be connected to system bus 703 via an appropriate port interface. In a networked environment, application programs 716 or program data 718 depicted relative to computer system 700, or portions thereof, may be stored in a remote memory storage device 740.

What have been described above are examples and embodiments of the devices and methods described and disclosed herein.

After having read and understood the present specification, those skilled in the art will now understand and appreciate that the various embodiments described herein provide solutions to longstanding problems, both in the use of electrophysiological mapping systems and in the use of cardiac ablation systems.

## Claims

1. A system (10) for combined electrophysiological mapping and ablation of a patient's heart (16), comprising:
an external electrophysiological mapping system, EMS, comprising: (a) a plurality of surface electrical sensing electrodes (100) configured to acquire surface electrocardiogram, ECG, signals from at least portions of a patient's torso (14); (b) a data acquisition device (200) operably connected to the surface electrical sensing electrodes and configured to condition the ECG signals provided thereby; (c) at least one non-transitory computer readable medium storing instructions executable by at least one processor (400) to perform a method for receiving and processing the ECG signals to provide on a display or a monitor a real-time or near-real-time voxel-model-derived visual representation or image of at least a portion of the patient's heart during a combined electrophysiological mapping and cardiac ablation procedure carried out on the patient;
a cardiac ablation system (500) comprising a catheter (512) configured for insertion inside the heart of the patient, the catheter comprising a distal end comprising a tissue ablation device configured to controllably form scar tissue on the patient's endocardium during the combined electrophysiological mapping and cardiac ablation procedure;
wherein the EMS is further programmed and configured to:
process the ECG signals during the combined electrophysiological mapping and cardiac ablation procedure to produce on the display or the monitor the real-time or near-real-time voxel-model-derived visual representation of one or more locations on the patient's heart where at least one scar has been created by the ablation device during the combined electrophysiological mapping and cardiac ablation procedure, the visual representation including maps (208) of electrical activity of the patient's heart (16);
and wherein the system (10) is further configured to:
monitor the patient's heart electrical activity while the cardiac ablation is carried out, monitoring electrophysiological changes in the heart by comparing the maps (208) of electrical activity over time; wherein the system (10) is further configured to:
generate a reference map of activation isochrones;
after receipt of a trigger signal from the cardiac ablation system (500) indicative of the start of the ablation, monitoring ablation-related changes between a current updated map of activation isochrones and a reference map of activation isochrones in a repeat loop, said changes being related to scar formation; and
send a respective trigger signal to the cardiac ablation system (500) after a difference between a current updated map of activation isochrones and a reference map of activation isochrones has crossed a threshold value , the respective trigger signal being indicative that a change related to scar formation has been detected.

2. The combined electrophysiological mapping and ablation system of claim 1, wherein the system (10) is configured to produce maps of electrophysiological changes in the heart, by computing the deviation of current ECG signals from reference ECG signals.

3. The combined electrophysiological mapping and ablation system of claim 1, wherein the visual representation of the one or more locations on the patient's heart where at least one scar has been created is based upon a velocity field or gradient or an amplitude field or gradient, the field or gradient being calculated by the EMS.

4. The combined electrophysiological mapping and ablation system of claim 1 or 3, wherein the cardiac ablation system (500) further comprises an electrical stimulation electrode located near or at the distal end of the catheter (512), the electrical stimulation electrode being configured to stimulate electrically intracardiac tissue of the patient (12) to produce an evoked response therein, the EMS being configured to detect ECG signals corresponding to the evoked response and process such signals to provide or refine the visual representation of the intracardiac location where scarring created by the ablation device has occurred.

5. The combined electrophysiological mapping and ablation system of claim 4, wherein a location of the distal end of the catheter (512) in the patient's heart (16) is provided in the visual representation on the basis of a point of origin of the evoked response being calculated by the EMS.

6. The combined electrophysiological mapping and ablation system of claim 1 or any of claims 3-5, wherein the EMS and the cardiac ablation system (500) are together configured to control a power level or duty cycle of the ablation delivered by the ablation device to the patient's heart (16), the power level or duty cycle being based on an amount, degree or extent of scarring of the patient's heart, which the EMS has determined at least partially to have occurred.

7. The combined electrophysiological mapping and ablation system of claim 1 or any of claims 3-6, wherein the EMS and the cardiac ablation system (500) are together configured to control an amount of time during which ablation is delivered by the ablation device to the patient's heart (16), the amount of time being based on an amount, degree or extent of scarring of the patient's heart, which the EMS has determined at least partially to have occurred.

8. The combined electrophysiological mapping and ablation system of claim 1 or any of claims 3-7, wherein the catheter (512) further comprises near or at its distal end at least one electrode, coil, sensor, transducer, magnetic source, or antenna that in combination with the EMS is configured to permit a location of the catheter's distal tip within the patient's heart (16) to be determined and displayed on the monitor or display in real-time or near-real-time.

9. The combined electrophysiological mapping and ablation system of claim 1 or any of claims 3-8, wherein the catheter (512) further comprises near or at its distal end at least one electrical sensing electrode configured to sense electrical signals generated by the heart (16), the cardiac ablation system (500) being configured to provide the electrical signals sensed thereby to the EMS as input signals thereto.

10. The combined electrophysiological mapping and ablation system of claim 1 or any of claims 3-9, wherein the tissue ablation device is a cryogenic ablation device, a radiofrequency ablation device, an ultrasound ablation device, a high-intensity focused ultrasound device, a chemical ablation device, or a laser ablation device.

11. A non-invasive system (10) for combined electrophysiological mapping and ablation of a patient's heart (16), comprising:
an external electrophysiological mapping system, EMS, comprising: (a) a plurality of surface electrical sensing electrodes (100) configured to acquire surface electrocardiogram, ECG, signals from at least portions of a patient's torso (14); (b) a data acquisition device (200) operably connected to the surface electrical sensing electrodes and configured to condition the ECG signals provided thereby; (c) at least one non-transitory computer readable medium storing instructions executable by at least one processor (400) to perform a method for receiving and processing the ECG signals to produce on a display or a monitor a real-time or near-real-time visual voxel-model-derived representation or image of at least a portion of the patient's heart during a combined electrophysiological mapping and cardiac ablation procedure carried out on the patient;
an external non-invasive cardiac ablation system (500) comprising at least one external directionally controllable and focusable source of ablation energy, the ablation energy source being configured to controllably form scar tissue on the patient's endocardium during the combined electrophysiological mapping and cardiac ablation procedure;
wherein the EMS is further programmed and configured to:
process the ECG signals during the combined electrophysiological mapping and cardiac ablation procedure to produce on the display or the monitor a real-time or near-real-time visual representation of one or more locations on the patient's heart where at least one scar has been created by the ablation device during the combined electrophysiological mapping and cardiac ablation procedure, the visual representation including maps (208) of electrical activity of the heart;
wherein the system (10) is further configured to:
monitor the patient's heart electrical activity while the cardiac ablation is carried out, monitoring electrophysiological changes in the heart, by comparing the maps (208) of electrical activity over time,
wherein the system (10) is further configured to:
generate a reference map of activation isochrones;
after receipt of a trigger signal from the cardiac ablation system (500) indicative of the start of the ablation, monitoring ablation-related changes between a current updated map of activation isochrones and a reference map of activation isochrones in a repeat loop, said changes being related to scar formation; and
send a respective trigger signal to the cardiac ablation system (500) after a difference between a current updated map of activation isochrones and a reference map of activation isochrones has crossed a threshold value, the trigger signal being indicative that a change has been detected.

12. The combined non-invasive electrophysiological mapping and ablation system of claim 11, wherein the system (10) is configured to produce maps of electrophysiological changes in the heart, by computing the deviation of current ECG signals from reference ECG signals.

13. The combined non-invasive electrophysiological mapping and ablation system of claim 11, wherein the visual representation of the one or more locations on the patient's heart where at least one scar has been created is based upon a velocity field or gradient or an amplitude field or gradient, an electrical conductivity field or gradient, the field or gradient being calculated by the EMS.

14. The combined non-invasive electrophysiological mapping and ablation system of claim 11 or 13, wherein the ablation energy source of the cardiac ablation system is included in a high intensity focused ultrasound (HIFU) system, a proton beam radiotherapy system, or an X-ray beam radiotherapy system.

15. The combined non-invasive electrophysiological mapping and ablation system of claim 11 or any of claims 13 to 14, further comprising one of: a magnetic resonance imaging and guiding system; a computed tomography imaging and guiding system; an ultrasound imaging and guiding system; the imaging and guiding system (300) configured to generate a three-dimensional image of at least a portion of the patient's heart (16) and to guide the location of the ablation energy that is applied to the patient's heart during the combined electrophysiological mapping and cardiac ablation procedure.

16. The combined non-invasive electrophysiological mapping and ablation system of claim 15, wherein the imaging and guiding system (300) and the EMS are together configured to produce on the display or monitor the real-time or near-real-time visual representation or image of at least a portion of the patient's heart (16) and the location of the ablation energy applied to the patient's heart.

17. The combined non-invasive electrophysiological mapping and ablation system of claim 11 or any of claims 13 to 16, wherein the EMS and the cardiac ablation system (500) are together configured to control a power level or duty cycle of the ablation delivered by the ablation device to the patient's heart (16), the power level or duty cycle being based on an amount, degree or extent of scarring of the patient's heart, which the EMS has determined at least partially to have occurred.

18. The combined non-invasive electrophysiological mapping and ablation system of claim 11 or any of claims 13 to 17, wherein the EMS and the cardiac ablation system (500) are together configured to control an amount of time during which ablation is delivered by the ablation device to the patient's heart (16), the amount of time being based on an amount, degree or extent of scarring of the patient's heart, which the EMS has determined at least partially to have occurred.

## Patentansprüche

1. System (10) für kombinierte elektrophysiologische Kartierung bzw. Mapping und Ablation eines Patientenherzens (16), umfassend:
ein externes elektrophysiologisches Kartierungs- bzw. Mappingsystem, EMS, umfassend: (a) eine Mehrzahl elektrischer Oberflächenmesselektroden (100), die ausgelegt sind, Oberflächenelektrokardiogramm-(EKG)-Signale von zumindest Abschnitten eines Patiententorsos (14) zu erfassen; (b) eine Datenerfassungsvorrichtung (200), die bedienbar mit den elektrischen Oberflächenmesselektroden verbunden ist und ausgelegt ist, die dadurch bereitgestellten EKG-Signale zu konditionieren bzw. aufzubereiten; (c) zumindest ein nicht vorübergehendes, computerlesbares Medium, das Anweisungen speichert, die von zumindest einem Prozessor (400) ausführbar sind, um ein Verfahren zum Erhalten und Verarbeiten der EKG-Signale auszuführen, um auf einem Display oder einem Monitor eine vom Voxelmodell abgeleitete visuelle Echtzeit- oder Fast-Echtzeit-Darstellung oder ein Bild von zumindest einem Abschnitt des Patientenherzens während eines am Patienten ausgeführten kombinierten elektrophysiologischen Mapping- und Herzablationseingriffs bereitzustellen;
ein Herzablationssystem (500), umfassend einen Katheter (512), der ausgelegt ist, in das Herz des Patienten eingeführt zu werden, wobei der Katheter ein Distalende umfasst, das eine Gewebeablationsvorrichtung umfasst, die ausgelegt ist, kontrollierbar während des kombinierten elektrophysiologischen Mapping- und Herzablationseingriffs Narbengewebe an dem Endokard des Patienten zu bilden;
wobei das EMS ferner programmiert und ausgelegt ist:
die EKG-Signale während des kombinierten elektrophysiologischen Mapping- und Herzablationseingriffs zu verarbeiten, um auf dem Display oder dem Monitor die vom Voxelmodell abgeleitete visuelle Echtzeit- oder Fast-Echtzeit-Darstellung von einer oder mehreren Stellen an dem Patientenherzen zu erzeugen, wo zumindest eine Narbe durch die Ablationsvorrichtung während des kombinierten elektrophysiologischen Mapping- und Herzablationseingriffs erzeugt wurde, wobei die visuelle Darstellung Abbildungen bzw. Karten (208) elektrischer Aktivität des Patientenherzens (16) enthält;
und wobei das System (10) ferner ausgelegt ist:
die elektrische Aktivität des Patientenherzens zu überwachen, während die Herzablation ausgeführt wird, wobei es elektrophysiologische Veränderungen in dem Herzen durch Vergleichen der Karten (208) elektrischer Aktivität über einen Zeitverlauf überwacht;
wobei das System (10) ferner ausgelegt ist:
eine Referenzkarte von Aktivierungsisochronen zu generieren;
nach Empfangen eines Auslösesignals von dem Herzablationssystem (500), das auf den Beginn der Ablation hinweist, ablationsbedingte Veränderungen zwischen einer aktuellen Karte von Aktivierungsisochronen und einer Referenzkarte von Aktivierungsisochronen in einer Wiederholungsschleife zu überwachen, wobei die Veränderungen narbenbildungsbedingt sind; und
ein jeweiliges Auslösesignal an das Herzablationssystem (500) zu senden, nachdem ein Unterschied zwischen einer aktuellen Karte von Aktivierungsisochronen und einer Referenzkarte von Aktivierungsisochronen einen Grenzwert überschritten hat, wobei das jeweilige Auslösesignal darauf hinweist, dass eine narbenbildungsbedingte Veränderung detektiert wurde.

2. System für kombiniertes elektrophysiologisches Mapping und Ablation nach Anspruch 1, wobei das System (10) ausgelegt ist, Karten elektrophysiologischer Veränderungen in dem Herzen zu erzeugen, indem es die Abweichung des aktuellen EKG-Signals von den Referenz-EKG-Signalen berechnet.

3. System für kombiniertes elektrophysiologisches Mapping und Ablation nach Anspruch 1, wobei die visuelle Darstellung von der einen oder den mehreren Stellen an dem Patientenherzen, wo zumindest eine Narbe erzeugt wurde, auf einem Geschwindigkeitsfeld oder -gefälle oder einem Amplitudenfeld oder -gefälle basiert, wobei das Feld oder das Gefälle von dem EMS berechnet ist.

4. System für kombiniertes elektrophysiologisches Mapping und Ablation nach Anspruch 1 oder 3, wobei das Herzablationssystem (500) ferner eine Elektrostimulationselektrode umfasst, die sich nahe dem oder an dem Distalende des Katheters (512) befindet, wobei die Elektrostimulationselektrode ausgelegt ist, intrakardiales Gewebe des Patienten (12) elektrisch zu stimulieren, um darin eine ausgelöste Reaktion bzw. ein evoziertes Potential zu erzeugen, wobei das EMS ausgelegt ist, EKG-Signale zu detektieren, die dem evozierten Potential entsprechen, und solche Signale zu verarbeiten, um die visuelle Darstellung der intrakardialen Stelle bereitzustellen oder zu verfeinern, wo durch die Ablationsvorrichtung erzeugte Vernarbung aufgetreten ist.

5. System für kombiniertes elektrophysiologisches Mapping und Ablation nach Anspruch 4, wobei eine Stelle des Distalendes des Katheters (512) in dem Patientenherzen (16) in der visuellen Darstellung auf der Basis eines von dem EMS berechneten Ausgangspunktes des evozierten Potentials bereitgestellt ist.

6. System für kombiniertes elektrophysiologisches Mapping und Ablation nach Anspruch 1 oder einem der Ansprüche 3-5, wobei das EMS und das Herzablationssystem (500) zusammen ausgelegt sind, einen Leistungspegel oder einen Arbeitszyklus der Ablation zu regeln bzw. zu steuern, der von der Ablationsvorrichtung an das Patientenherz (16) abgegeben wird, wobei der Leistungspegel oder der Arbeitszyklus auf dem Betrag, dem Grad oder dem Ausmaß der Vernarbung des Patientenherzens basiert, die laut Bestimmung des EMS zumindest teilweise aufgetreten ist.

7. System für kombiniertes elektrophysiologisches Mapping und Ablation nach Anspruch 1 oder einem der Ansprüche 3-6, wobei das EMS und das Herzablationssystem (500) zusammen ausgelegt sind, einen Zeitbetrag zu regeln bzw. zu steuern, während der die Ablation von der Ablationsvorrichtung an das Patientenherz (16) abgegeben wird, wobei der Zeitbetrag auf dem Betrag, dem Grad oder dem Ausmaß der Vernarbung des Patientenherzens basiert, die laut Bestimmung des EMS zumindest teilweise aufgetreten ist.

8. System für kombinierte elektrophysiologisches Mapping und Ablation nach Anspruch 1 oder einem der Ansprüche 3-7, wobei der Katheter (512) ferner nahe seinem oder an seinem Distalende zumindest eine Elektrode, eine Spule, einen Sensor, einen Wandler, eine magnetische Quelle oder eine Antenne umfasst, die bzw. der in Kombination mit dem EMS ausgelegt ist, zu erlauben, dass eine Stelle der Distalspitze des Katheters innerhalb des Patientenherzens (16) bestimmt und auf dem Monitor oder Display in Echtzeit oder Fast-Echtzeit angezeigt wird.

9. System für kombiniertes elektrophysiologisches Mapping und Ablation nach Anspruch 1 oder einem der Ansprüche 3-8, wobei der Katheter (512) ferner nahe seinem oder an seinem Distalende zumindest eine elektrische Messelektrode umfasst, die ausgelegt ist, von dem Herzen (16) generierte elektrische Signale zu messen, wobei das Herzabalationssystem (500) ausgelegt ist, die dadurch gemessenen elektrischen Signale dem EMS als Eingangssignale dafür bereitzustellen.

10. System für kombiniertes elektrophysiologisches Mapping und Ablation nach Anspruch 1 oder einem der Ansprüche 3-9, wobei die Gewebeablationsvorrichtung eine Kryoablationsvorrichtung, eine Hochfrequenzablationsvorrichtung, eine Ultraschallablationsvorrichtung, eine Vorrichtung für hochintensiven fokussierten Ultraschall, eine Chemieablationsvorrichtung oder eine Laserablationsvorrichtung ist.

11. Nicht-invasives System (10) für kombinierte elektrophysiologische Kartierung bzw. Mapping und Ablation eines Patientenherzens (16), umfassend:
ein externes elektrophysiologisches Kartierungs- bzw. Mappingsystem, EMS, umfassend: (a) eine Mehrzahl elektrischer Oberflächenmesselektroden (100), die ausgelegt sind, Oberflächenelektrokardiogramm-(EKG)-Signale von zumindest Abschnitten eines Patiententorsos (14) zu erfassen; (b) eine Datenerfassungsvorrichtung (200), die bedienbar mit den elektrischen Oberflächenmesselektroden verbunden ist und ausgelegt ist, die dadurch bereitgestellten EKG-Signale zu konditionieren bzw. aufzubereiten; (c) zumindest ein nicht vorübergehendes, computerlesbares Medium Anweisungen speichert, die von zumindest einem Prozessor (400) ausführbar sind, um ein Verfahren zum Erhalten und Verarbeiten der EKG-Signale auszuführen, um auf einem Display oder einem Monitor eine vom Voxelmodell abgeleitete visuelle Echtzeit- oder Fast-Echtzeit-Darstellung oder ein Bild von zumindest einem Abschnitt des Patientenherzens während eines am Patienten ausgeführten kombinierten elektrophysiologischen Mapping- und Herzablationseingriffs bereitzustellen;
ein externes, nicht-invasives Herzablationssystem (500), umfassend zumindest eine externe, direktional regel- bzw. steuerbare und fokussierbare Quelle der Ablationsenergie, wobei die Ablationsenergiequelle ausgelegt ist, kontrollierbar während des kombinierten elektrophysiologischen Mapping- und Herzablationseingriffs Narbengewebe an dem Endokard des Patienten zu bilden;
wobei das EMS ferner programmiert und ausgelegt ist:
die EKG-Signale während des kombinierten elektrophysiologischen Mapping- und Herzablationseingriffs zu verarbeiten, um auf dem Display oder dem Monitor visuelle Echtzeit- oder Fast-Echtzeit-Darstellung von einer oder mehreren Stellen an dem Patientenherzen zu erzeugen, wo zumindest eine Narbe Herzablationseingriffs durch die Ablationsvorrichtung während des kombinierten elektrophysiologischen Mapping- und Herzablationseingriffs erzeugt wurde, wobei die visuelle Darstellung Abbildungen bzw. Karten (208) elektrischer Aktivität des Herzens enthält;
wobei das System (10) ferner ausgelegt ist:
die elektrische Aktivität des Patientenherzens zu überwachen, während die Herzablation ausgeführt wird, wobei es elektrophysiologische Veränderungen in dem Herzen durch Vergleichen der Karten (208) elektrischer Aktivität über einen Zeitverlauf überwacht;
wobei das System (10) ferner ausgelegt ist:
eine Referenzkarte von Aktivierungsisochronen zu generieren;
nach Empfangen eines Auslösesignals von dem Herzablationssystem (500), das auf den Beginn der Ablation hinweist, ablationsbedingte Veränderungen zwischen einer aktuellen Karte von Aktivierungsisochronen und einer Referenzkarte von Aktivierungsisochronen in einer Wiederholungsschleife zu überwachen, wobei die Veränderungen narbenbildungsbedingte sind; und
ein jeweiliges Auslösesignal an das Herzablationssystem (500) zu senden, nachdem ein Unterschied zwischen einer aktuellen Karte von Aktivierungsisochronen und einer Referenzkarte von Aktivierungsisochronen einen Grenzwert überschritten hat, wobei das Auslösesignal darauf hinweist, dass eine Veränderung detektiert wurde.

12. System für kombiniertes nicht-invasives elektrophysiologisches Mapping und Ablation nach Anspruch 11, wobei das System (10) ausgelegt ist, Karten elektrophysiologischer Veränderungen in dem Herzen zu erzeugen, indem es die Abweichung des aktuellen EKG-Signals von den Referenz-EKG-Signalen berechnet.

13. System für kombiniertes nicht-invasives elektrophysiologisches Mapping und Ablation nach Anspruch 11, wobei die visuelle Darstellung von der einen oder den mehreren Stellen an dem Patientenherzen, wo zumindest eine Narbe erzeugt wurde, auf einem Geschwindigkeitsfeld oder -gefälle oder einem Amplitudenfeld oder -gefälle basiert, wobei das Feld oder Gefälle von dem EMS berechnet ist.

14. System für kombiniertes nicht-invasives elektrophysiologisches Mapping und Ablation nach Anspruch 11 oder 13, wobei die Ablationsenergiequelle des Herzablationssystems in einem hochintensiven-fokussierten-Ultraschall (HIFU)-System, einem Protonenstrahlentherapiesystem oder einem Röntgenstrahlentherapiesystem enthalten ist.

15. System für kombiniertes nicht-invasives elektrophysiologisches Mapping und Ablation nach Anspruch 11 oder einem der Ansprüche 13 bis 14, ferner umfassend eines aus: einem Magnetresonanz-Bildgebungs- und Führungssystem; einem Computertomographie- und Führungssystem; einem Ultraschall-Bildgebungs- und Führungssystem; wobei das Bildgebungs- und Führungssystem (300) ausgelegt ist, ein dreidimensionales Bild von zumindest einem Abschnitt des Patientenherzens (16) zu generieren und die Stelle der Ablationsenergie zu führen, die während des kombinierten elektrophysiologischen Mapping- und Herzablationseingriffs auf das Patientenherz angewendet wird.

16. System für kombiniertes nicht-invasives elektrophysiologisches Mapping und Ablation nach Anspruch 15, wobei das Bildgebungs- und Führungssystem (300) und das EMS zusammen ausgelegt sind, auf dem Display oder Monitor die visuelle Echtzeit- oder Fast-Echtzeit-Darstellung oder das -Bild von zumindest einem Abschnitt des Patientenherzens (16) und der Stelle der auf das Patientenherz angewendeten Ablationsenergie zu erzeugen.

17. System für kombiniertes nicht-invasives elektrophysiologisches Mapping und Ablation nach einem der Ansprüche 13 bis 16, wobei das EMS und das Herzablationssystem (500) zusammen ausgelegt sind, einen Leistungspegel oder einen Arbeitszyklus der Ablation zu regeln bzw. zu steuern, der von der Ablationsvorrichtung an das Patientenherz (16) abgegeben wird, wobei der Leistungspegel oder der Arbeitszyklus auf dem Betrag, dem Grad oder dem Ausmaß der Vernarbung des Patientenherzens basiert, die laut Bestimmung des EMS zumindest teilweise aufgetreten ist.

18. System für kombiniertes nicht-invasives elektrophysiologisches Mapping und Ablation nach Anspruch 11 oder einem der Ansprüche 13 bis 17, wobei das EMS und das Herzablationssystem (500) zusammen ausgelegt sind, einen Zeitbetrag zu regeln bzw. zu steuern, während der die Ablation von der Ablationsvorrichtung an das Patientenherz (16) abgegeben wird, wobei der Zeitbetrag auf dem Betrag, dem Grad oder dem Ausmaß der Vernarbung des Patientenherzens basiert, die laut Bestimmung des EMS zumindest teilweise aufgetreten ist.

## Revendications

1. Système (10) pour la cartographie électrophysiologique et l'ablation du cœur d'un patient combinées (16), comprenant :
un système de cartographie électrophysiologique externe, EMS (Electrophysiological Mapping System), comprenant: (a) une pluralité d'électrodes de détection électrique de surface (100) configurées pour acquérir un électrocardiogramme de surface, ECG, des signaux d'au moins des parties du torse d'un patient (14); (b) un dispositif d'acquisition de données (200) connecté fonctionnellement aux électrodes de détection électrique de surface et configuré pour traiter les signaux ECG ainsi fournis; (c) au moins un support lisible par ordinateur non transitoire stockant des instructions exécutables par au moins un processeur (400) pour exécuter un procédé de réception et de traitement des signaux ECG pour fournir sur un écran ou un moniteur une représentation visuelle ou une image dérivée du modèle voxel en temps réel ou quasi réel d'au moins une partie du cœur du patient pendant une cartographie électrophysiologique et une procédure d'ablation cardiaque combinées effectuées sur le patient;
un système d'ablation cardiaque (500) comprenant un cathéter (512) configuré pour être inséré à l'intérieur du cœur du patient, le cathéter comprenant une extrémité distale comprenant un dispositif d'ablation tissulaire configuré pour former de manière contrôlable du tissu cicatriciel sur l'endocarde du patient pendant la cartographie électrophysiologique et la procédure d'ablation cardiaque combinées;
dans lequel l'EMS est en outre programmé et configuré pour:
traiter les signaux ECG pendant la procédure combinée de cartographie électrophysiologique et d'ablation cardiaque pour produire sur l'écran ou le moniteur la représentation visuelle dérivée du modèle de voxel en temps réel ou quasi réel d'un ou plusieurs emplacements sur le cœur du patient où au moins une cicatrice a été créée par le dispositif d'ablation au cours de la procédure combinée de cartographie électrophysiologique et d'ablation cardiaque, la représentation visuelle comprenant des cartes (208) de l'activité électrique du cœur du patient (16);
et dans lequel le système (10) est en outre configuré pour:
surveiller l'activité électrique cardiaque du patient pendant l'ablation cardiaque, surveiller les changements électrophysiologiques dans le cœur en comparant les cartes (208) de l'activité électrique dans le temps;
dans lequel le système (10) est en outre configuré pour:
générer une carte de référence des isochrones d'activation;
après réception d'un signal de déclenchement du système d'ablation cardiaque (500) indiquant le début de l'ablation, surveiller les changements liés à l'ablation entre une carte actualisée des isochrones d'activation et une carte de référence des isochrones d'activation dans une boucle de répétition, lesdits changements étant liés à la formation de cicatrices; et
envoyer un signal de déclenchement respectif au système d'ablation cardiaque (500) après qu'une différence entre une carte actualisée des isochrones d'activation et une carte de référence des isochrones d'activation ait franchi une valeur de seuil, le signal de déclenchement respectif indiquant qu'un changement lié à la formation de cicatrices a été détecté.

2. Système combiné de cartographie et d'ablation électrophysiologique selon la revendication 1, dans lequel le système (10) est configuré pour produire des cartes des changements électrophysiologiques dans le cœur, en calculant l'écart des signaux ECG actuels par rapport aux signaux ECG de référence.

3. Système combiné de cartographie électrophysiologique et d'ablation selon la revendication 1, dans lequel la représentation visuelle d'un ou plusieurs emplacements sur le cœur du patient où au moins une cicatrice a été créée est basée sur un champ ou gradient de vitesse ou un champ ou gradient d'amplitude, le champ ou gradient étant calculé par l'EMS.

4. Système combiné de cartographie électrophysiologique et d'ablation selon la revendication 1 ou 3, dans lequel le système d'ablation cardiaque (500) comprend en outre une électrode de stimulation électrique située près de ou à l'extrémité distale du cathéter (512), l'électrode de stimulation électrique étant configurée pour stimuler électriquement le tissu intracardiaque du patient (12) pour y produire un potentiel évoqué, l'EMS étant configuré pour détecter des signaux ECG correspondants au potentiel évoqué et pour traiter de tels signaux de manière à fournir ou à affiner la représentation visuelle de l'emplacement intracardiaque où les lésions cicatricielles créées par le dispositif d'ablation se sont produites.

5. Système combiné de cartographie électrophysiologique et d'ablation selon la revendication 4, dans lequel un emplacement de l'extrémité distale du cathéter (512) dans le cœur du patient (16) est fourni dans la représentation visuelle sur la base d'un point d'origine du potentiel évoqué calculé par l'EMS.

6. Système combiné de cartographie électrophysiologique et d'ablation selon la revendication 1 ou l'une des revendications 3 à 5, dans lequel l'EMS et le système d'ablation cardiaque (500) sont configurés ensemble pour contrôler un niveau de puissance ou un rapport cyclique de l'ablation délivré par le dispositif d'ablation au cœur du patient (16), le niveau de puissance ou le rapport cyclique étant basé sur une quantité, un degré ou une étendue de lésions cicatricielles du cœur du patient, que l'EMS a déterminé au moins partiellement comme s'étant produit.

7. Système combiné de cartographie électrophysiologique et d'ablation selon la revendication 1 ou l'une des revendications 3 à 6, dans lequel l'EMS et le système d'ablation cardiaque (500) sont configurés ensemble pour contrôler une durée pendant laquelle l'ablation est délivrée par le dispositif d'ablation au cœur du patient (16), la durée étant basée sur une quantité, un degré ou une étendue de lésions cicatricielles du cœur du patient, que l'EMS a déterminé au moins partiellement comme s'étant produit.

8. Système combiné de cartographie électrophysiologique et d'ablation selon la revendication 1 ou l'une des revendications 3 à 7, dans lequel le cathéter (512) comprend en outre près de ou à son extrémité distale au moins une électrode, une spire, un capteur, un transducteur, une source magnétique ou une antenne qui, en combinaison avec l'EMS, est configuré pour permettre de déterminer et d'afficher en temps réel ou quasi réel un emplacement de la pointe distale du cathéter dans le cœur du patient (16).

9. Système combiné de cartographie électrophysiologique et d'ablation selon la revendication 1 ou l'une des revendications 3 à 8, dans lequel le cathéter (512) comprend en outre près de ou à son extrémité distale au moins une électrode de détection électrique configurée pour détecter les signaux électriques générés par le cœur (16), le système d'ablation cardiaque (500) étant configuré pour fournir les signaux électriques ainsi détectés à l'EMS en tant que signaux d'entrée.

10. Système combiné de cartographie électrophysiologique et d'ablation selon la revendication 1 ou l'une des revendications 3 à 9, dans lequel le dispositif d'ablation tissulaire est un dispositif d'ablation cryogénique, un dispositif d'ablation par radiofréquence, un dispositif d'ablation par ultrasons, un dispositif à ultrasons focalisé à haute intensité, un dispositif d'ablation chimique ou un dispositif d'ablation laser.

11. Système non invasif (10) pour cartographie électrophysiologique et ablation combinées du cœur d'un patient (16), comprenant :
un système de cartographie électrophysiologique externe, EMS, comprenant: (a) une pluralité d'électrodes de détection électrique de surface (100) configurées pour acquérir un électrocardiogramme de surface, ECG, des signaux d'au moins des parties du torse d'un patient (14); (b) un dispositif d'acquisition de données (200) connecté fonctionnellement aux électrodes de détection électrique de surface et configuré pour traiter les signaux ECG ainsi fournis; (c) au moins un support lisible par ordinateur non transitoire stockant des instructions exécutables par au moins un processeur (400) pour exécuter un procédé de réception et de traitement des signaux ECG pour fournir sur un écran ou un moniteur une représentation visuelle ou une image dérivée du modèle voxel en temps réel ou quasi réel d'au moins une partie du cœur du patient pendant une cartographie électrophysiologique et une procédure d'ablation cardiaque combinées effectuées sur le patient;
un système d'ablation cardiaque non invasif externe (500) comprenant au moins une source d'énergie d'ablation externe directionnellement contrôlable et focalisable, la source d'énergie d'ablation étant configurée pour former de manière contrôlable du tissu cicatriciel sur l'endocarde du patient pendant la cartographie électrophysiologique et la procédure d'ablation cardiaque combinées; dans lequel l'EMS est en outre programmé et configuré pour:
traiter les signaux ECG pendant la cartographie électrophysiologique et la procédure d'ablation cardiaque combinées pour produire sur l'écran ou le moniteur une représentation visuelle en temps réel ou quasi réel d'un ou plusieurs emplacements sur le cœur du patient où au moins une cicatrice a été créée par le dispositif d'ablation pendant la procédure combinée de cartographie électrophysiologique et d'ablation cardiaque, la représentation visuelle comprenant des cartes (208) de l'activité électrique du cœur;
dans lequel le système (10) est en outre configuré pour:
surveiller l'activité électrique cardiaque du patient pendant l'ablation cardiaque, en surveillant les changements électrophysiologiques dans le cœur, en comparant les cartes (208) de l'activité électrique dans le temps, dans lequel le système (10) est en outre configuré pour:
générer une carte de référence des isochrones d'activation;
après réception d'un signal de déclenchement du système d'ablation cardiaque (500) indiquant le début de l'ablation, surveiller les changements liés à l'ablation entre une carte actualisée des isochrones d'activation et une carte de référence des isochrones d'activation dans une boucle de répétition, lesdits changements étant liés à la formation de cicatrices; et
envoyer un signal de déclenchement respectif au système d'ablation cardiaque (500) après qu'une différence entre une carte actualisée des isochrones d'activation et une carte de référence des isochrones d'activation ait franchi une valeur de seuil, le signal de déclenchement indiquant qu'un changement a été détecté.

12. Système combiné de cartographie et d'ablation électrophysiologique non invasif selon la revendication 11, dans lequel le système (10) est configuré pour produire des cartes des changements électrophysiologiques dans le cœur, en calculant l'écart des signaux ECG en cours par rapport aux signaux ECG de référence.

13. Système combiné de cartographie et d'ablation électrophysiologique non invasif selon la revendication 11, dans lequel la représentation visuelle d'un ou plusieurs emplacements sur le cœur du patient où au moins une cicatrice a été créée est basée sur un champ ou gradient de vitesse ou un champ ou gradient d'amplitude, un champ ou gradient de conductivité électrique, le champ ou gradient étant calculé par l'EMS.

14. Système combiné de cartographie et d'ablation électrophysiologique non invasif selon la revendication 11 ou 13, dans lequel la source d'énergie d'ablation du système d'ablation cardiaque est incluse dans un système à ultrasons focalisés de haute intensité (HIFU), un système de radiothérapie à faisceau de protons ou un système de radiothérapie par rayons X.

15. Système combiné de cartographie et d'ablation électrophysiologique non invasif selon la revendication 11 ou l'une des revendications 13 à 14, comprenant en outre l'un des points suivants: un système d'imagerie et de guidage par résonance magnétique; un système d'imagerie et de guidage par tomodensitométrie; un système d'imagerie et de guidage par ultrasons; système d'imagerie et de guidage (300) configuré pour générer une image tridimensionnelle d'au moins une partie du cœur du patient (16) et pour guider l'emplacement de l'énergie d'ablation qui est appliquée au cœur du patient pendant la cartographie électrophysiologique et la procédure d'ablation cardiaque combinées.

16. Système combiné de cartographie et d'ablation électrophysiologique non invasif selon la revendication 15, dans lequel le système d'imagerie et de guidage (300) et l'EMS sont configurés ensemble pour produire sur l'écran ou le moniteur la représentation visuelle ou l'image en temps réel ou quasi réel d'au moins une partie du cœur du patient (16) et l'emplacement de l'énergie d'ablation appliquée au cœur du patient.

17. Système combiné de cartographie et d'ablation électrophysiologique non invasif selon la revendication 11 ou l'une des revendications 13 à 16, dans lequel l'EMS et le système d'ablation cardiaque (500) sont configurés ensemble pour contrôler un niveau de puissance ou un rapport cyclique de l'ablation délivrée par le dispositif d'ablation du cœur du patient (16), le niveau de puissance ou le rapport cyclique étant basé sur une quantité, un degré ou une étendue de lésions cicatricielles du cœur du patient, que l'EMS a déterminé au moins partiellement comme s'étant produit.

18. Système combiné de cartographie et d'ablation électrophysiologique non invasif selon la revendication 11 ou l'une des revendications 13 à 17, dans lequel l'EMS et le système d'ablation cardiaque (500) sont configurés ensemble pour contrôler une durée pendant laquelle l'ablation est délivrée par le dispositif d'ablation du cœur du patient (16), la durée étant basée sur une quantité, un degré ou une étendue de lésions cicatricielles du cœur du patient, que l'EMS a déterminé au moins partiellement comme s'étant produit.
